# EUROPÄISCHE PATENTSCHRIFT

(11) **EP 2 165 770 B1**
(45) Veröffentlichungstag und Bekanntmachung des Hinweises auf die Patenterteilung: **16.12.2015**
(21) Anmeldenummer: 09011576.7
(22) Anmeldetag: 10.09.2009
(51) Int. Cl.: B05B 13/00

(54) **Austragvorrichtung**
Discharge device
Dispositif de sortie

(30) Priorität: 19.09.2008 US 284242
(43) Veröffentlichungstag der Anmeldung: 24.03.2010
(73) Patentinhaber: Aptar Radolfzell GmbH, 78315 Radolfzell (DE)
(72) Erfinder: Carter, Miro, Daytona Beach Florida 32124 (US)
(74) Vertreter: Patentanwälte Ruff, Wilhelm, Beier, Dauster & Partner mbB

(56) Entgegenhaltungen:
- DE-A1-102007 048 651
- US-A1- 2005 100 655

## Beschreibung

Die Erfindung betrifft einen Spender zum Austrag eines flüssigen pharmazeutischen Mediums in oder auf den Körper eines Benutzers mit einem Reservoir zur Aufbewahrung des Mediums, einer Druckerzeugungseinrichtung zur Druckbeaufschlagung des Mediums, die als Verdrängerpumpe mit einer volumenveränderbare Druckkammer sowie einer Pumpenbetätigungseinrichtung auf, mittels derer das Druckkammervolumen beeinflusst werden kann, ausgebildet ist, einer Auslassöffnung zum Austragen des Mediums in oder auf den Körper eines Benutzers und einem Auslassventil mit einem Ventilsitz und einem zwischen einer Öffnungsstellung und einer Schließstellung relativ zum Ventilsitz beweglichen Ventilkörper.

Die Erfindung betrifft weiterhin ein Verfahren zum Austragen eines flüssigen pharmazeutischen Mediums mittels eines Spenders sowie ein Herstellungsverfahren zur Herstellung eines Spenders.

Gattungsgemäße Spender sind aus dem Stand der Technik in vielfältiger Form bekannt. Sie dienen dem Austrag pharmazeutischer Medien, wobei unter pharmazeutischen Medien im Zusammenhang mit dieser Erfindung jegliche Substanzen verstanden werden, die zu medizinischen Zwecken in oder auf dem Körper eines Benutzers ausgebracht werden. Insbesondere sind derartige Spender zur nasalen oder ophtalmischen Anwendung verwendbar.

Gattungsgemäße Spender weisen ein Reservoir auf, in dem das pharmazeutische Medium zunächst gelagert ist. Aus diesem Reservoir wird ein Teilvolumen des Mediums zum Zwecke des Austrags gefördert und mittels einer Druckerzeugungseinrichtung unter Druck gesetzt. Das unter Druck gesetzte Medium wird dann durch eine Auslassöffnung in oder auf das zu behandelnde Körperteil des Benutzers abgegeben. Das gattungsgemäße vorgesehene Auslassventil wird zumeist verwendet, um das in der Druckerzeugungseinrichtung druckbeaufschlagte Medium zunächst davon abzuhalten, durch die Auslassöffnung zu entweichen. Erst bei Erreichen bestimmter Randbedingungen, insbesondere bei Erreichen eines konstruktiv bedingten Austragdrucks, wird das Auslassventil geöffnet, um den Zugang zur Auslassöffnung zu gewährleisten, so dass das Teilvolumen des Mediums dann ausgetragen wird.

Die Dosierung des auszutragenden Mediums wird bei gattungsgemäßen Spendern in der Regel über die Druckerzeugungseinrichtung bestimmt. Die Druckerzeugungseinrichtung beaufschlagt das konstruktiv durch die Maße der Druckerzeugungseinrichtung vorgegebenes Teilvolumen des Mediums mit Druck. Dieses Teilvolumen wird bei jedem Austragvorgang ausgegeben. Das Auslassventil selbst beeinflusst das ausgetragene Volumen dabei nicht, sondern dient lediglich der Gewährleistung einer gewünschten Austragcharakteristik, indem es erst ab einem bestimmten Flüssigkeitsdruck im Medium öffnet.

Problematisch an derartigen Ausgestaltungen ist es, dass selbst bei hohen Standards des Herstellungsverfahrens Toleranzen an der Druckerzeugungseinrichtung zu variierenden Austragsvolumina führen. Dieser Effekt wird umso gravierender, je geringer das auszutragende Volumen ist, da sich die unvermeidbaren Ungenauigkeiten dann umso stärker auswirken.

Aus der US 2005/0100655 A1 ist eine Beschichtungsanlage bekannt, bei der ein spulengesteuerter Austragkopf vorgesehen ist. Aus der DE 10 2007 048651 A1 ist ebenfalls eine Beschichtungseinrichtung bekannt. Diese verfügt über ein Magnetventil zur Steuerung der Zufuhr von Druckluft.

### Aufgabe und Lösung

Aufgabe der Erfindung ist es daher, einen gattungsgemäßen Spender derart weiterzubilden, dass der Austrag des Mediums und insbesondere das ausgetragene Volumen besser beeinflusst werden können. Aufgabe ist es weiterhin, ein Verfahren zum Austrag eines Mediums bereitzustellen, welches eine höhere Beeinflussbarkeit der Austragscharakteristik gewährleistet.

Erfindungsgemäß wird zum Erreichen dieser Aufgabe ein gattungsgemäßer Spender um eine elektrische Ventilbetätigungseinrichtung ergänzt, mittels derer der Ventilkörper mittelbar durch einen elektrischen Ventilsteuerstrom kraftbeaufschlagbar ist.

Die Ventilbetätigungseinrichtung ist demnach dazu ausgebildet, den Ventilkörper gegenüber dem Ventilsitz mit einer Kraft zu beaufschlagen, wobei diese Kraft vorzugsweise über den elektrischen Ventilsteuerstrom dosierbar ist.

Das Auslassventil, welches dadurch über die Ventilbetätigungseinrichtung beeinflussbar ist, ist erfindungsgemäß in einem Bereich des Spenders vorgesehen, dessen Inhalt durch die Druckbeaufschlagungseinrichtung druckbeaufschlagt werden kann. Das Auslassventil kann im einfachsten Falle lediglich dem Zweck dienen, während einer Inbetriebnahme des Spenders eine Entlüftung dieses Bereichs zu erlauben, indem es geöffnet wird, bevor mittels der Druckerzeugungsvorrichtung die Luft durch das Ventil hindurch herausgedrückt wird. Insbesondere umfasst ist jedoch eine Ausgestaltung, bei der das Auslassventil zwischen der Druckerzeugungsvorrichtung und der Auslassöffnung vorgesehen ist und somit den Zustrom des Mediums zur Auslassöffnung steuern kann.

Die Druckerzeugungseinrichtung in Form einer Verdrängerpumpe ist dafür ausgebildet, aus dem Reservoir mit Medium gespeist zu werden. Besonders vorteilhaft ist die Verwendung einer Kolbenpumpe oder einer Balgpumpe, wobei die Pumpen vorzugsweise jeweils derart ausgebildet sind, dass bei einer Verringerung des Druckkammervolumens zunächst die Verbindung mit dem Reservoir unterbrochen wird, um anschließend die Druckbeaufschlagung des in der Druckkammer befindlichen Mediums durchzuführen. Als Pumpenbetätigungseinrichtung im Zusammenhang mit dieser Erfindung werden jene Komponenten angesehen, die mittelbar die Volumenveränderung der Druckkammer herbeiführen. Im Falle einer Kolbenpumpe handelt es sich bei der Pumpenbetätigungseinrichtung um die Bauteile, mittels derer der Kolben zur Verringerung des Druckkammervolumens kraftbeaufschlagt werden kann.

Vorzugsweise tritt das Medium unmittelbar in Form einzelner Tröpfchen aus, die durch das Auslassventil durch schnelles Öffnen und Schließen gebildet werden. Dies ist für ophtalmische Anwendungen ideal. Zur Herstellung eines anwendungsspezifisch optimalen Sprühbildes kann jedoch auch eine angepasste Strömungsgeometrie an der Auslassöffnung gewählt werden, die ein eine Verwirbelung, eine Vernebelung oder ähnliches bewirkt und für topische, nasale oder orale Anwendungen von Vorteil ist.

Die elektrische Ventilbetätigungseinrichtung ist dabei vorzugsweise derart ausgebildet, dass sie den Ventilkörper ohne weitere Kraftbeaufschlagungsmittel in seine Öffnungsstellung und seine Schließstellung überführen kann. Dies erlaubt es, alleine mittels eines entsprechenden Steuergeräts, welches zur Steuerung der Ventilbetätigungseinrichtung ausgebildet ist, den Austragvorgang zu beginnen und zu beenden. Somit kann durch gezielte Ansteuerung der Ventilbetätigungseinrichtung ein Volumen des Mediums ausgetragen werden, welches wesentlich geringer als das durch die Druckerzeugungseinrichtung druckbeaufschlagte Medium ist. Die Ungenauigkeit hinsichtlich des von der Druckerzeugungseinrichtung geförderten Volumens, welches druckbeaufschlagt wird, schlägt sich daher nicht unmittelbar im ausgetragenen Volumen des Mediums nieder, da dieses ausgetragene Volumen lediglich vom Öffnung und Schließen des Auslassventils abhängt, nicht aber von dem druckbeaufschlagten Volumen. Die elektrische Ventilbetätigungseinrichtung erlaubt darüber hinaus auch unübliche Austragscharakteristiken, beispielsweise einen gepulsten Austrag, bei dem durch wiederholtes Öffnen und Schließen des Auslassventils in schneller Folge einzelne Tropfen des Mediums abgegeben werden, sowie einen Austrag mit verringertem Austragsdruck, bei dem das Auslassventil zur Herstellung einer Drosselwirkung nur geringfügig geöffnet wird. Ein weiterer Anwendungszweck für einen Spender mit einer erfindungsgemäßen elektrische Ventilbetätigungseinrichtung liegt darin, dass der Auslassdruck gezielt beeinflusst werden kann, wodurch es möglich wird, Spender für verschiedene Zwecke baulich identisch zu gestalten und lediglich in Hinblick auf die Ansteuerung der Ventilbetätigungseinrichtung spezifisch anzupassen.

Das Auslassventil kann derart ausgebildet sein, dass eine Bewegung des Ventilkörpers lediglich über die elektrische Ventilbetätigungseinrichtung möglich ist. Bei einer solchen Ausgestaltung kann der Ventilkörper aus seiner vorzugsweise geschlossenen Ruhelage nur durch die Ventilbetätigungseinrichtung entfernt werden. Andere Einflussparameter wie der Flüssigkeitsdruck des Mediums können lediglich dadurch einbezogen werden, dass sie von einem Steuergerät mittels entsprechender Sensorik erfasst werden und dieses Steuergerät in Abhängigkeit von diesen Parametern das Auslassventil mittels der Ventilbetätigungseinrichtung öffnet bzw. schließt. Die elektrische Ventilbetätigungseinrichtung kann jedoch auch als eines von mehreren Einflussmitteln zur Kraftbeaufschlagung des Ventilkörpers ausgestaltet sein. Mit einer solchen Ausgestaltung wird es möglich, durch die elektrische Ventilbetätigungseinrichtung die anderen Einflussmittel zu ergänzen, beispielsweise um eine Feinjustierung der Austragscharakteristik vorzunehmen.

Das Auslassventil kann derart ausgebildet sein, dass der Ventilkörper durch den Flüssigkeitsdruck des Mediums in einer dem Auslassventil zugeordneten Ventilkammer in Richtung seiner Öffnungsstellung kraftbeaufschlagbar ist.

Das Medium in der Ventilkammer ist bei dieser Ausgestaltung mittels einer Druckfläche am Ventilkörper in der Lage, bei ausreichend hohem Flüssigkeitsdruck den Ventilkörper in Richtung seiner Öffnungsstellung zu verlagern. Damit kann ein Öffnen des Auslassventils unabhängig von einer Aktivierung der elektrischen Ventilbetätigungseinrichtung erfolgen, indem das Medium unter Druck den Ventilkörper in einer Öffnungsstellung drückt. Dies kann mit der elektrischen Ventilbetätigungseinrichtung vorteilhaft kombiniert werden, beispielsweise indem der Öffnungsvorgang alleine oder vorwiegend durch den Flüssigkeitsdruck erzielt wird und ein nachfolgender Schließzustand nach dem Medienaustrag durch die elektrische Ventilbetätigungseinrichtung gegen den Flüssigkeitsdruck in der Ventilkammer erzielt wird. Dies erlaubt es, in aus dem Stand der Technik bekannter Art und Weise den Austragvorgang bei Erreichen eines vorgegebenen Mindestdrucks zu beginnen und ihn nach einer Austragszeitspanne enden zu lassen, obwohl der Flüssigkeitsdruck zu diesem Zeitpunkt unverändert oberhalb dieses Öffnungsdrucks ist. Im Zusammenhang mit einem durch Flüssigkeitsdruck in seinen Öffnungszustand überführbaren Auslassventil kann die Verwendung des Ventilbetätigungsmittels auch vorteilhaft dazu verwendet werden, den Öffnungsdruck durch eine Kraftbeaufschlagung des Ventilkörpers im geschlossenen Zustand zu verändern. So kann erreicht werden, dass das Auslassventil erst bei einem sehr hohen oder bereits bei einem sehr geringen Druck in der Ventilkammer in den Öffnungszustand gelangt.

Insbesondere zur Aufrechterhaltung des Schließzustandes des Auslassventils ist dem Auslassventil vorzugsweise eine Ventilfedereinrichtung zugeordnet, mittels derer der Ventilkörper in Richtung seiner Schließstellung mit einer Federkraft beaufschlagbar ist. Im Zusammenhang mit dieser Erfindung wird als Federeinrichtung dabei jedes ohne externe Energiezufuhr arbeitende Kraftbeaufschlagungsmittel angesehen, mittels dessen eine permanente Kraftbeaufschlagung zweier Bauteile relativ zueinander möglich ist. Die Kraftbeaufschlagung kann beispielsweise über mit identischen Polen aufeinander zu orientierten Permanentmagneten erreicht werden. Insbesondere von Vorteil ist jedoch die Verwendung einer mechanischen Feder, insbesondere einer Schraubenfeder, durch die der Ventilkörper kraftbeaufschlagt wird. Die Ventilfedereinrichtung gewährleistet, dass in einem nicht genutzten Ruhezustand das Auslassventil mit der Austragvorrichtung in seiner Schließstellung verbleibt. Vorzugsweise wird der Ventilkörper von der Ventilfedereinrichtung mit einem mittleren Druck von mindestens 5 MPa an einem umlaufenden Kontaktbereich gegen den Ventilsitz gedrückt, um dadurch eine wirksame Keimbarriere zu schaffen. Die Ventilfedereinrichtung kann insbesondere im Zusammenspiel mit dem oben beschriebenen Auslassventil verwendet werden, welches unmittelbar durch den Flüssigkeitsdruck in der Ventilkammer in einen Öffnungszustand überführbar ist. Die Ventilfedereinrichtung ist dabei maßgeblich für den erforderlichen Öffnungsdruck des Mediums verantwortlich. Die Ventilbetätigungseinrichtung kann vorteilhaft genutzt werden, um Abweichungen der Federkraft von einer Soll-Federkraft zu korrigieren. Dabei kann die von Spender zu Spender variierende Federkraft der Ventilfedereinrichtung durch Ergänzung um die in gleiche oder entgegengesetzte Richtung wirkende Kraft der Ventilbetätigungseinrichtung auf ein zumindest in der relevanten Auslenkungslage der Ventilfedereinrichtung einheitliches Maß gebracht werden.

Was die Ausgestaltung der elektrischen Ventilbetätigungseinrichtung angeht, so kann diese zur Bewegung des Ventilkörpers im einfachsten Falle einen Elektromotor oder einen elektrischen Linearaktuator umfassen. Diese können beispielsweise unter Zwischenschaltung eines Getriebes oder eines anderweitigen Umsetzers mit dem Ventilkörper wirkgekoppelt sein.

Als besonders vorteilhaft wird jedoch ein Spender angesehen, bei der die elektrische Ventilbetätigungseinrichtung eine zur Erzeugung eines Magnetfeldes durch den elektrischen Steuerstrom vorgesehene Betätigungsspule sowie einen gegenüber der Ventilbetätigungsspule relativ beweglichen und mittels des Magnetfeldes gegenüber der Ventilbetätigungsspule kraftbeaufschlagten Gegenkörper aufweist, wobei der Gegenkörper oder die Ventilbetätigungsspule ortsfest zum Ventilkörper ausgebildet ist.

Durch diese Ausgestaltung kann eine Relativbewegung des Gegenkörpers zur Ventilbetätigungsspule unter Nutzung der Lorentz-Kraft erreicht werden. Durch die Festlegung des Gegenkörpers oder der Ventilbetätigungsspule am Ventilkörper kann der Ventilkörper mit der Lorentz-Kraft beaufschlagt werden. Abhängig von der Stromrichtung in der Spule sind dabei Kraftbeaufschlagungen sowohl in Richtung der Schließstellung als auch in Richtung der Öffnungsstellung möglich. Durch Festlegung der Stromstärke kann die Kraftbeaufschlagung und/oder die Verlagerung des Ventilkörpers präzise festgelegt werden. Der Gegenkörper ist vorzugsweise als Permanentmagnet oder alternativ als Elektromagnet ausgebildet, um die Wirkung des Spulenstroms zu erhöhen. Insbesondere kann ein kunststoffgebundener Magnetwerkstoff Anwendung finden. Dies erlaubt es sogar, den Gegenkörper und den Ventilkörper einstückig zu gestalten. Der Gegenkörper kann fest mit dem Ventilkörper verbunden ist, während die Spule ortsfest zum Ventilsitz angeordnet ist und den Gegenkörper zumindest teilweise umgibt. Alternativ dazu kann die Ventilbetätigungsspule ortsfest am Ventilkörper vorgesehen sein und sich gegenüber einem ortsfesten permanentmagnetischen Gegenkörper im Zuge der Strombeaufschlagung der Ventilbetätigungsspule bewegen. Diese zweite Variante entspricht bezüglich ihrer Funktionsweise einem elektrodynamischen Lautsprecher. Ihr Vorteil liegt insbesondere in der aufgrund der geringen Spulenmasse erzielbaren geringen Masse des Ventilkörpers, die ein besonders schnelles Öffnen und Schließen des Ventils gestattet.

Eine Alternative zur Ausgestaltung der Ventilbetätigungseinrichtung mit Ventilbetätigungsspule sieht vor, dass die Ventilbetätigungseinrichtung einen durch den Ventilsteuerstrom steuerbaren Ventilpiezoaktuator zur Kraftbeaufschlagung des Ventilkörpers aufweist. Dieser Ventilpiezoaktuator ändert bei Beaufschlagung mit dem Ventilsteuerstrom seine Ausdehnung, was zur Verlagerung des Ventilkörpers genutzt werden kann. Vorzugsweise weist der Ventilpiezoaktuator einen Piezostapel auf, um eine große Auslenkung erreichbar zu machen. Da die Auslenkung eines Ventilpiezoaktuators verglichen mit der Auslenkung eines Gegenkörpers relativ zu einer Ventilbetätigungsspule klein ist, ist vorzugsweise zusätzlich eine Übertragungseinrichtung zur Wegverlängerung vorgesehen, beispielsweise ein schwenkbarer Hebel, der gehäusefest schwenkbar gelagert und einerseits am Ventilkörper und andererseits am Piezoaktuator festgelegt ist und über den die Ausdehnung des Piezoaktuators verstärkt an den Ventilkörper weitergegeben wird.

Bei einer Weiterbildung der Erfindung ist eine Ventilmesseinrichtung vorgesehen, die zur Erfassung einer Verlagerung des Ventilkörpers relativ zum Ventilsitz ausgebildet ist. Diese Ventilmesseinrichtung ist derart ausgestaltet, dass sie verbunden mit einer Auswerteelektronik erfassen kann, ob der Ventilkörper sich relativ zum Ventilsitz bewegt und/oder wo sich der Ventilkörper relativ zum Ventilsitz befindet. Die genaue Position des Ventilkörpers kann auch bei einer Ventilmesseinrichtung erfasst werden, die nur die Bewegung des Ventilkörpers wahrnimmt, indem ausgehend von einer bekannten Ausgangsposition eine Verlagerungszeit erfasst wird, die unter Einbeziehung der hinsichtlich ihrer Größe bekannten auf dem Ventilkörper wirkenden Kräfte auch eine Berechnung der Lage des Ventilkörpers gestattet.

Die Ventilmesseinrichtung kann im einfachsten Falle benutzt werden, um eine Funktionsüberprüfung des Spenders durchzuführen. Dabei wird mittels der Ventilbetätigungseinrichtung eine Verlagerung des Ventilkörpers bewirkt, wobei mittels der Ventilmesseinrichtung erfasst wird, ob die beabsichtigte Verlagerung stattgefunden hat. Darüber hinaus kann die Ventilmesseinrichtung dazu genutzt werden, um eine anderweitig bewirkte Bewegung des Ventilkörpers zu erfassen und gegebenenfalls zu unterstützen. So kann die Ventilmesseinrichtung eine Verlagerung des Ventilkörpers als Reaktion auf einen gestiegenen Flüssigkeitsdruck in der Ventilkammer erkennen und infolgedessen durch die Ventilbetätigungseinrichtung den Öffnungsvorgang des Ventils unterstützen und/oder durch die Ventilbetätigungseinrichtung nach Ablauf einer bestimmten Zeitspanne nach Öffnen des Ventils den Ventilkörper in seine Schließstellung verlagern.

Von besonderem Vorteil ist die Ventilmesseinrichtung zur Erfassung der spenderspezifischen Parameter hinsichtlich des Auslassventils. Zwar werden verschiedenen Spender gleichen Typs stets auf die gleiche Art und Weise hergestellt. Es kann jedoch dennoch zu uneinheitlichen Eigenschaften des Auslassventils kommen, beispielsweise durch Unterschiede hinsichtlich der Federkraft einer Ventilfedereinrichtung oder hinsichtlich von Reibungskräften, die einer Verlagerung des Ventilkörpers gegenüber dem Ventilsitz entgegenwirken. Diese für jeden Spender voneinander abweichenden Nebenkräfte, die in Richtung oder entgegen der Richtung der Kraftbeaufschlagung durch die Ventilbetätigungseinrichtung wirken, können mittels der Ventilmesseinrichtung erfasst werden. Je nach Masse des Ventilkörpers muss die Trägheitskraft den Ventilkörpers bei der Erfassung / Errechnung der Nebenkräfte mit einbezogen werden. Im einfachsten Falle werden die einzelnen Nebenkräfte, also insbesondere die Federkraft und die Reibungskraft, nicht einzeln, sondern lediglich als zusammengefasste Nebenkraft erfasst. Die Federkraft ist zwar an sich nicht konstant, kann für den üblicherweise geringen Hub des Ventilkörpers von vorzugsweise unter 0,5 mm, insbesondere von weniger als 0,2 mm, als konstant angenommen werden.

Es ist beispielsweise möglich, durch eine Messung der Auslenkung des Ventilkörpers bei einer definierten, der Federkraft entgegengesetzten Kraft die Eigenschaften der Federeinrichtung zu erfassen. Ebenfalls ist es möglich, durch eine Zeitmessung beim Übergang vom Öffnungszustand in den Schließzustand zu erfassen, wie groß die der Bewegung des Ventilkörpers entgegenwirkenden Nebenkräfte sind, da diese die Übergangszeit beeinflussen. Die Zeitmessung kann insbesondere herangezogen werden, um die mittlere Geschwindigkeit des Ventilkörpers beim Übergang vom Öffnungszustand in den Schließzustand oder umgekehrt zu berechnen, welche bei Kenntnis der hierfür aufgebrachten Leistung die Erfassung der Nebenkräfte, insbesondere der Reibungsund Federkräfte, gestattet. Da hierfür der zurückgelegte Weg bekannt sein muss, kann die Bewegung vorzugsweise zwischen zwei Anschlägen erfolgen, die die Bewegungsfreiheit des Ventilkörpers in der Öffnungs- und Schließstellung begrenzen.

Die Ventilmesseinrichtung wird vorzugsweise derart angesteuert, dass die Messung mehrfach hintereinander erfolgt, vorzugsweise mindestens dreimal hintereinander. Hierdurch können Messfehler und Schwankungen ausgeglichen werden. Durch Messungen bei der Bewegung des Ventilkörpers in gegenläufige Richtungen können die Federkraft und die Reibungskraft auch isoliert ermittelt werden.

Die durch die Ventilmesseinrichtung somit erfassbaren austragsvorrichtungsspezifischen Einflussfaktoren auf die Bewegung des Ventilkörpers können nach Erfassung durch die Ventilbetätigungseinrichtung kompensiert werden, so dass sich ein vorgegebenes Verhalten des Auslassventils einstellt. So kann eine Federkraft, die geringer als die Soll-Federkraft ist, dadurch ausgeglichen werden, dass mittels der Ventilbetätigungseinrichtung eine zusätzliche Kraftkomponente in Richtung der Schließstellung bewirkt wird. Dadurch kann der Öffnungsdruck des Mediums, bei dem der Ventilkörper sich in Richtung seiner Öffnungsstellung verlagert, präzise festgelegt werden. Die Anpassung der durch die Ventilbetätigungseinrichtung bewirkten Kraft in Abhängigkeit des Ergebnisses der Messung durch die Ventilmesseinrichtung kann anhand eines hinterlegten Formelwerks erfolgen. Alternativ dazu kann im Speicher des Spenders auch eine Tabelle hinterlegt sein, die beispielsweise das Ergebnis der genannten Zeitmessung einer durch die Ventilbetätigungseinrichtung zu bewirkenden Kraft zuordnet.

Alternativ zu einer solchen Kompensierung können die erfassten Werte bei einem Spender einbezogen werden, um beispielsweise die Öffnungszeit des Auslassventils zur Erreichung eines vorgegebenen Austragvolumens anzupassen. Dies wird im Folgenden noch näher erläutert.

Als besonders vorteilhaft wird eine Ventilmesseinrichtung angesehen, die eine Ventilmessspule aufweist, die zur Erfassung der Bewegung eines am Ventilkörper vorgesehenen Gegenkörpers ausgebildet ist. Bei einer solchen Ausgestaltung wird durch die Bewegung des vorzugsweise permanentmagnetischen Gegenkörpers in der Ventilmessspule induzierte Spannung herangezogen, um die Verlagerung des Gegenkörpers und damit des Ventilkörpers erfassen zu können. Die induzierte Spannung ist von dem Magnetfeld, der Ventilmessspule und der Geschwindigkeit des Gegenkörpers abhängig. Der Gegenkörpers ist vorzugsweise identisch mit einem Gegenkörper, der mittels einer Ventilbetätigungsspule zur Verlagerung des Ventilkörpers genutzt wird. Bei der Verwendung einer Messspule als Messeinrichtung ist es besonders vorteilhaft, wenn lediglich erfasst wird, ob die durch die Bewegung des Ventilkörpers induzierte Spannung größer Null ist, ob also eine Bewegung vorliegt. Die hieraus ableitbare Information, wie lange der Ventilkörper bewegt wird, erlaubt für sich genommen bereits Rückschlüsse auf die Nebenkräfte, ohne dass hierfür eine Auswertung der induzierten Spannung der Höhe nach erfolgen muss. Eine aufwändige Auswertung der Höhe der induzierten Spannung ist je nach Art der Auswertung entbehrlich. Wie auch die Ventilbetätigungsspule kann auch die Ventilmessspule ortsfest zum Gehäuse des Auslassventils oder Ortsfest zum Ventilkörper ausgebildet sein.

Alternative Gestaltungen der Ventilmesseinrichtung sehen vor, dass mittels eines Piezosensors die Verlagerung des Ventilkörpers erfasst wird.

Als besonders vorteilhaft wird es angesehen, wenn die Ventilbetätigungseinrichtung zur Kraftbeaufschlagung des Ventilkörpers mit einer Maximalkraft ausgebildet ist, die größer als die in Richtung der Schließstellung wirkende Federkraft. Diese Gestaltung erlaubt es, das Auslassventil gegen die Federkraft in den Öffnungszustand zu überführen, auch ohne dass in der Ventilkammer ein den Ventilkörper in die Öffnungsstelle drückender Flüssigkeitsdruck gegeben ist. Dies ermöglicht insbesondere, das Auslassventil alleine durch die Ventilbetätigungseinrichtung zu öffnen, um nach dem Herstellungsvorgang im Strömungspfad zwischen Druckerzeugungseinrichtung und Auslassöffnung vorhandene Luft zu entfernen. Hierzu wird das Auslassventil geöffnet und eine Druckbeaufschlagung der Luft durchgeführt, um die Luft durch das Auslassventil hindurch aus dem Strömungspfad hinauszudrücken.

Bei einer besonders bevorzugten Weiterbildung der Erfindung ist zwischen der Druckerzeugungseinrichtung und dem Auslassventil ein Durchflussbegrenzer vorgesehen.

Als Durchflussbegrenzer wird im Zusammenhang mit dieser Erfindung ein Abschnitt des Flüssigkeitspfades zwischen der Druckerzeugungseinrichtung und der Auslassöffnung angesehen, der einen gegenüber den anderen Komponenten auf dem Flüssigkeitspfad höheren Strömungswiderstand bietet. Der Durchflussbegrenzer weist hierzu vorzugsweise eine Verengung auf oder ist als ein dünner Kanal ausgebildet, durch den bei den bestimmungsgemäß im Spender herrschenden Drücken beidseitig des Durchflussbegrenzers nur ein begrenzter Volumenstrom hindurchströmen kann, wobei dieser Volumenstrom der auszutragenden Menge des Mediums entspricht. Der Durchflussbegrenzer bildet im Flüssigkeitspfad zwischen Druckerzeugungsvorrichtung und Auslassöffnung einen Strömungswiderstand, der mindestens 50%, vorzugsweise mindestens 80%, insbesondere vorzugsweise mindestens 90%, des Gesamtströmungswiderstandes zwischen Druckerzeugungseinrichtung und Auslassöffnung darstellt. Dies kann insbesondere durch einen besonders kleinen freien Querschnitt erzielt werden, da die Querschnittsfläche in den Strömungswiderstand des Durchflussbegrenzers quadratisch eingeht. Die Querschnittsfläche beträgt vorzugsweise weniger als 1 mm², vorzugsweise weniger als 0,5 mm². Durch die Wahl einer geeigneten besonders einfachen Geometrie, beispielsweise durch die Ausgestaltung des Durchflussbegrenzers als dünner Verbindungskanal, oder durch die Ermittlung des Strömungswiderstandes durch Versuche und Messungen, kann der Strömungswiderstand des Durchflussbegrenzers leicht berechnet oder ermittelt werden. Da der Volumenstrom durch den Durchflussbegrenzer proportional zu der Druckdifferenz zwischen den beiden Enden des Durchflussbegrenzers ist, kann bei bekanntem Strömungswiderstand und bekannten Differenzdruck berechnet werden, welcher Volumenstrom, also welches Flüssigkeitsvolumen pro Zeiteinheit, durch den Durchflussbegrenzer hindurchströmt. Durch eine Gestaltung, bei der der Strömungswiderstand des Durchflussbegrenzers verantwortlich ist für den überwiegenden Teil des Strömungswiderstandes von der Druckerzeugungsvorrichtung bis zur Auslassöffnung, ist gewährleistet, dass das anhand der besagten Druckdifferenz berechenbare Volumen des Mediums, das den Durchflussbegrenzer in einer bestimmten Zeit durchströmt, dem ausgetragenen Volumen entspricht.

In Kenntnis des Strömungswiderstandes des Durchflussbegrenzers sowie in Kenntnis der Drücke beidseits des Durchflussbegrenzers kann der Volumenstrom durch den Durchflussbegrenzer berechnet werden. Wenn gewährleistet ist, dass die Druckverhältnisse beidseitig des Durchflussbegrenzers sich während eines Austragvorgangs nicht wesentlich ändern, kann eine vorgegebene Dosierung des ausgetragenen Mediums sehr genau anhand eines angepassten Öffnungszeitraums des Auslassventils erzielt werden.

Besonders bevorzugt ist eine Ausgestaltung, bei der eine der Druckerzeugungseinrichtung zugeordnete Druckkammer, deren Volumen durch die Druckerzeugungseinrichtung verminderbar ist, und eine dem Auslassventil zugeordnete Ventilkammer vorgesehen sind, wobei der Durchflussbegrenzer zwischen der Druckkammer und der Ventilkammer vorgesehen ist.

Die Druckkammer und die Ventilkammer eignen sich besonders gut zur Herstellung definierter Drücke, die es gestatten, den Volumenstrom anhand der Druckdifferenz, der Viskosität des Mediums und der Geometrie des Durchflussbegrenzers exakt festzulegen. Im Falle der Druckkammer kann durch eine entsprechende Gestaltung der Druckerzeugungseinrichtung gewährleistet werden, dass das Medium in der Druckkammer stets mit dem gleichen Druck beaufschlagt wird. Im Falle der Ventilkammer kann ebenfalls ein definierter Druck hergestellt werden, wobei dies besonders vorteilhaft dadurch geschieht, dass das Auslassventil zum Öffnen bei genau diesem Flüssigkeitsdruck ausgebildet ist. Hierdurch ist gewährleistet, dass der vorgegebene Druck in der Ventilkammer nicht überschritten wird. Um ein Öffnen des Auslassventils in Abhängigkeit des Flüssigkeitsdrucks zu gewährleisten, muss der durch die Druckerzeugungseinrichtung in der Druckkammer erzeugte Druck höher sein als der konstruktiv bedingte Öffnungsdruck des Auslassventils. Sowohl für die Druckkammer als auch für die Ventilkammer gilt, dass die jeweils bei einem Austrag dort herrschenden Drücke zusätzlich durch die Ventilbetätigungseinrichtung bzw. eine Pumpenbetätigungseinrichtung beeinflusst werden können, um eine vorab definierte Druckdifferenz beim Austrag herzustellen.

Neben einer Gestaltung, bei der durch konstruktive Maßnahmen definierte Drücke in der Druckkammer und der Ventilkammer hergestellt werden, ist es auch möglich, durch eine gezielte Berechnung oder Erfassung des aktuellen Drucks in der Druckkammer und/oder der Ventilkammer abzuschätzen, wie groß die Druckdifferenz ist und diese Druckdifferenz bei der Berechnung der Öffnungszeit des Auslassventils heranzuziehen. Auch hierdurch kann eine exakte Abschätzung des Volumenstroms durch den Durchflussbegrenzer hindurch erfolgen.

Bei einer Weiterbildung der Erfindung weist der Durchflussbegrenzer einen Verbindungskanal mit zumindest abschnittsweise einheitlichem Querschnitt auf. Diese Gestaltung ist besonders einfach herzustellen und erlaubt anhand des Gesetzes von Hagen-Poiseuille eine besonders einfache Berechnung des Strömungswiderstandes. Vorteilhaft ist ein derartiger Verbindungskanal jedoch auch, da er einfach herzustellen ist und da er besonders gut eine laminare Strömung des Mediums gewährleistet, so dass die Proportionalität zwischen Volumenstrom und Druckdifferenz beidseitig des Durchflussbegrenzers gegeben ist. Der Abschnitt mit einheitlichem Querschnitt ist dabei derart ausgebildet, dass er mindestens 80% des Strömungswiderstandes des Durchflussbegrenzers verursacht. Die Querschnittfläche des Verbindungskanals liegt vorzugsweise unterhalb von 1 mm², insbesondere vorzugsweise unterhalb von 0,1 mm².

Besonders einfach ist eine Ausgestaltung, bei der die Pumpenbetätigungseinrichtung als manuell betätigbare Pumpenbetätigungseinrichtung ausgebildet ist. Die Druckbeaufschlagung des Mediums in der Druckkammer erfolgt demzufolge mit einer Energie, die von einem Benutzer manuell aufgebracht wird. Dies erlaubt besonders einfache und kostengünstige Gestaltungen. Vorzugsweise weist die Pumpenbetätigungseinrichtung ein Pumpenfedermittel auf, welches zunächst manuell spannbar ist und welches derart ausgebildet ist, dass es nach einer Auslösung im Zuge seiner Entspannung eine Volumenverringerung der Druckkammer bewirkt. Durch eine derartige Ausgestaltung mit manuell gespanntem Pumpenfedermittel ist eine benutzerunabhängige Druckbeaufschlagung des Mediums in der Druckkammer möglich. Die vom Benutzer aufgebrachte Energie wird dabei nicht unmittelbar und sofort zur Druckbeaufschlagung des Mediums verwendet. Sie wird stattdessen zunächst als Federenergie im Pumpenfedermittel gespeichert, um nach der Auslösung ohne weiteres Zutun des Benutzers zur Druckbeaufschlagung des Mediums in der Druckkammer genutzt zu werden.

Besonders vorteilhaft ist es dabei, wenn der Spannvorgang des Pumpenfedermittels durch die Bewegung einer Komponente des Spenders erfolgt, welche zur Überführung des Spenders in einen Nutzzustand ohnehin zu bewegen ist. Beispielsweise kann eine verschwenkbare oder anderweitig gegenüber dem Gehäuse des Spenders bewegliche Schutzkappe vorgesehen sein, die vor der Benutzung des Spenders zu öffnen ist und die im Zuge des Öffnens durch eine entsprechende Wirkkopplung zu einem Spannen des Pumpenfedermittels führt.

Alternativ zu einer manuell betätigbaren Pumpenbetätigungseinrichtung kann die Pumpenbetätigungseinrichtung auch als elektrische Pumpenbetätigungseinrichtung ausgebildet sein, die zur Veränderung des Druckkammervolumens mittels eines elektrischen Pumpenstroms ausgebildet ist.

Die Pumpenbetätigung mit einer elektrischen Pumpenbetätigungseinrichtung erlaubt insbesondere eine reproduzierbare Druckbeaufschlagung des Mediums. Die Druckbeaufschlagung kann durch eine Kraft bewirkt werden, die aufgrund der elektrischen Verursachung genau dosierbar ist. Besonders bevorzugt ist eine Ausgestaltung, bei der die elektrische Pumpenbetätigungseinrichtung zur Verlagerung eines Kolbens vorgesehen ist, der die Druckkammer begrenzt.

Wie auch bei dem Ventilkörper des Auslassventils kann die elektrische Betätigungsvorrichtung durch einen Elektromotor oder einen Pumpenpiezoaktuator realisiert sein, wobei vorzugsweise mittels eines Getriebes oder eines Hebels die Kraftübertragung vom Motor oder vom Piezoaktuator auf die Druckerzeugungsvorrichtung bzw. deren Kolben erfolgt. Als besonders vorteilhaft wird jedoch eine elektrische Pumpenbetätigungseinrichtung angesehen, die eine zur Erzeugung eines Magnetfelds durch den elektrischen Pumpensteuerstrom vorgesehener Pumpenbetätigungsspule und ein gegenüber der Pumpenbetätigungsspule relativ bewegliches und mittels des Magnetfeldes gegenüber der Pumpenbetätigungsspule kraftbeaufschlagbares Gegenelement aufweist. Dabei ist das vorzugsweise permanentmagnetische Gegenelement oder die Pumpenbetätigungsspule derart ausgebildet und/oder angeordnet, dass das Druckkammervolumen durch eine Relativbewegung des Gegenelements zur Pumpenbetätigungsspule veränderbar ist.

Wie auch bei der Ausgestaltung des Auslassventils mit elektrischer Ventilbetätigungseinrichtung ist auch bei der Pumpenbetätigungsspule vorzugsweise vorgesehen, dass das Gegenelement zur Veränderung des Druckkammervolumens beweglich ausgebildet ist, während die Pumpenbetätigungsspule ortsfest ausgebildet ist. Alternativ kann jedoch auch hier vorgesehen sein, dass die Pumpenbetätigungsspule beweglich ausgebildet ist und beispielsweise am Kolben der Druckerzeugungsvorrichtung festgelegt ist, während das Gegenelement feststehend angeordnet ist.

Bei einer Weiterbildung der Erfindung ist eine Pumpenmesseinrichtung vorgesehen, die zur Erfassung des Zustandes der Pumpenbetätigungseinrichtung ausgebildet ist. Diese Pumpenmesseinrichtung kann wie auch die Ventilmesseinrichtung derart ausgebildet sein, dass sie erfasst, ob gegenwärtig eine Volumenveränderung der Druckkammer, beispielsweise durch eine Kolbenbewegung, stattfindet. Sie kann jedoch auch derart ausgebildet sein, dass sie die Lage eines Kolbens einer Kolbenpumpe oder den Kompressionszustand des Balgs einer Balgpumpe erfasst. Die Pumpenmesseinrichtung ist derart ausgebildet, dass sie mittels elektrischer Signale die erfassten Daten an ein Steuergerät weitergeben kann. Der Zweck der Pumpenmesseinrichtung liegt wie auch bei der Ventilmesseinrichtung im einfachsten Falle in der Möglichkeit, eine Funktionsüberprüfung stattfinden zu lassen. Weiterhin erlaubt es die Pumpenmesseinrichtung, Nebenkräfte wie beispielsweise Reibungskräfte, die bei der Druckbeaufschlagung des Mediums der Kraft der Pumpenbetätigungseinrichtung entgegenwirken, zu erfassen, um die entsprechenden Kräfte durch die Pumpenbetätigungseinrichtung auszugleichen oder bei der Berechnung eines Öffnungszeitraums mit einzubeziehen. Ein besonderer Vorteil beim Vorliegen einer Pumpenmesseinrichtung ergibt sich daraus, dass diese genutzt werden kann, um den Erstbefüllungszustand des Spenders zu überprüfen. Hierunter ist die Befüllung des Flüssigkeitspfades vom Reservoir bis zur Austragöffnung zu verstehen. Da ein bestimmungsgemäßer Austragvorgang mit definierter Austragsmenge erst möglich ist, wenn die Luft aus dem druckbeaufschlagten Bereich entfernt wurde, wird bei gattungsgemäßen Spendern üblicherweise zunächst durch verschiedenste Maßnahmen die Luft aus diesen Bereichen entfernt. Die Pumpenmesseinrichtung ermöglicht eine Überprüfung dieses Zustandes, indem bei geschlossenem Auslassventil das Volumen der Druckkammer vermindert wird. Wenn eine derartige Volumenverminderung jedoch aufgrund des nicht kompressiblen Charakters des Mediums nicht mehr möglich ist, so ist dies ein Zeichen dafür, dass sämtliche Luft entwichen ist.

Die Pumpenmesseinrichtung kann eine Pumpenmessspule aufweisen, die zur Erfassung der Lage eines der Pumpenbetätigungseinrichtung zugeordneten Bauteils ausgebildet ist, welches zum Zweck der Beeinflussung des Druckkammervolumens lageveränderlich ist. Die Pumpenmessspule entspricht bezüglich ihrer Funktionsweise der Ventilmessspule. Sie erlaubt die Erfassung der Relativstellung der Pumpenmessspule zu einem Gegenelement, welches beispielsweise an einem im Zuge der Verringerung des Druckkammervolumens lageveränderlichen Kolbens festgelegt ist. Dieses Element ist vorzugsweise als Permanentmagnet ausgebildet.

Hinsichtlich der Pumpenbetätigungsvorrichtung und der Pumpenmesseinrichtung sind die oben in Hinblick auf die Ventilbetätigungseinrichtung und Ventilmesseinrichtung beschriebenen Vorteile und Weiterbildungen ebenfalls gültig, soweit sie sich nicht auf spezifische Eigenschaften des Auslassventils beziehen.

Bei einer Weiterbildung der Erfindung ist in der Druckkammer und/oder in der Ventilkammer ein Drucksensor vorgesehen. Ein solcher Drucksensor erlaubt die unmittelbare Erfassung des Drucks in der jeweiligen Kammer. Im Falle eines Drucksensors in der Ventilkammer kann dies beispielsweise genutzt werden, um die elektrische Ventilbetätigungseinrichtung zum Öffnen des Auslassventils zu veranlassen, sobald ein definierter Grenzdruck überschritten ist. In Kombination mit dem oben beschriebenen Durchflussbegrenzer erlauben die Drucksensoren eine präzise Bestimmung des Flüssigkeitsvolumens, welches durch den Durchflussbegrenzer hindurchgeströmt. Selbst bei sich ändernden Drücken in der Druckkammer bzw. der Ventilkammer kann der Volumenstrom, der sich hierdurch ebenfalls ändert, permanent überwacht werden und damit das ausgetragene Gesamtvolumen berechnet werden. Die Drucksensoren können verwendet werden, um die Ventilmesseinrichtung sowie die Pumpenmesseinrichtung hinsichtlich der Aufgabe, Nebenkräfte am Ventilkörper oder am Kolben zu erfassen, zu ersetzen, da die Kenntnis dieser Nebenkräfte bei direkter Messung der Drücke nicht zwingend erforderlich ist. Es sind jedoch auch Gestaltungen umfasst, bei denen sowohl Messeinrichtungen am Ventil und/oder der Pumpe vorgesehen sind und zusätzlich Drucksensoren zur Überwachung des Drucks in der Druckkammer und/oder der Ventilkammer vorgesehen sind. Bei solchen Gestaltungen können die verschiedenen Ergebnisse der Messeinrichtungen und Drucksensoren verwendet werden, um besonders zuverlässige Informationen über die jeweils herrschenden Drücke zu erhalten. Verwendbare Drucksensoren umfassen beispielsweise piezoelektrische Drucksensoren, kapazitive Drucksensoren und induktive Drucksensoren.

Bei einer Weiterbildung der Erfindung weist der Spender einen Durchflusssensor auf, der vorzugsweise zwischen der Druckkammer und der Ventilkammer angeordnet ist. Der Durchflusssensor ermittelt die ausgetragene Flüssigkeitsmenge. Dies erlaubt es, nach Erreichen eines gewünschten Austragvolumens das Auslassventil durch die Ventilbetätigungseinrichtung zu schließen. Neben der Anordnung zwischen Druckkammer und Ventilkammer kann die Flüssigkeitsstrommesseinrichtung auch zwischen dem Auslassventil und der Auslassöffnung angeordnet sein.

Vorzugsweise weist ein erfindungsgemäßer Spender ein Steuergerät auf, durch das der Ventilsteuerstrom und/oder der Pumpensteuerstrom steuerbar sind und/oder durch das Ausgangssignale der Pumpenmesseinrichtung und/oder der Ventilmesseinrichtung auswertbar sind. Das Steuergerät dient primär der Auswertung der im Spender vorgesehenen Sensoren und Messeinrichtungen sowie der Betätigung der elektrischen Ventilbetätigungseinrichtung und/oder der elektrischen Pumpenbetätigungseinrichtung. Das Steuergerät ist dabei vorzugsweise zur Durchführung der nachfolgend noch beschriebenen Arbeitsverfahren ausgebildet.

Weiterhin ist das Steuergerät vorzugsweise dazu ausgebildet, die Austragvorgänge zu zählen, so dass jederzeit über den Füllstand des Reservoirs Daten vorliegen. Vorzugsweise ist das Steuergerät weiterhin mit Eingabe- und Ausgabemitteln verbunden, beispielsweise einem Display zur Ausgabe von Statusmeldungen und/oder Tastern und zur Erfassung von Steuerbetätigungen wie einer Startbetätigung, mit der der Austragvorgang ausgelöst wird. Das Steuergerät ist vorzugsweise derart ausgebildet, dass es einen Austragvorgang mittels der Pumpenbetätigungseinrichtung und/oder der Ventilbetätigungseinrichtung durchführen kann.

Dabei speichert das Steuergerät vorzugsweise die Zeit des Austrags, um nachfolgend für eine vorgegebene Sperrzeit einen weiteren Austragvorgang zu verhindern. Das Steuergerät kann insbesondere auch benutzt werden, um Daten über die durchgeführten Austragvorgänge in einem Speicher zu hinterlegen, aus dem sie später zur Überprüfung der Benutzung des Spenders auslesbar sind.

Die Erfindung betrifft weiterhin ein Verfahren zum Austragen eines flüssigen pharmazeutischen Mediums mittels eines Spenders genannter Art, wobei ein Austragvorgang mit drei Schritten stattfindet. Zunächst wird das Medium innerhalb einer Druckkammer einer Druckerzeugungseinrichtung mit einem Druck p1 beaufschlagt. Anschließend wird ein Auslassventil zum Zweck des Medienaustrags geöffnet. Nach Verstreichen einer Öffnungszeit Δt wird das Auslassventil durch ein elektrisches Steuersignal wieder geschlossen, um den Medienaustrag zu beenden.

Die Besonderheit bei diesem Austragverfahren liegt darin, dass das Auslassventil durch ein elektrisches Steuersignal geschlossen wird. Als elektrisches Steuersignal im Zusammenhang mit diesem Verfahren wird eine Änderung eines Steuerstroms verstanden, die zur Steuerung des Auslassventils verwendet wird. Das Schließen des Auslassventils per Steuersignal kann demzufolge auch durch den Wegfall eines zuvor angelegten Steuerstroms erfolgen. Durch das gezielte Schließen des Auslassventils ist es möglich, ein Volumen des Mediums auszutragen, welches geringer sein kann als das in der Druckkammer druckbeaufschlagte Volumen. Das Auslassventil schließt nicht erst, wenn das druckbeaufschlagte Volumen vollständig oder nahezu vollständig ausgetragen wurde und der Druck des Mediums infolgedessen sinkt, sondern sobald das elektrische Steuersignal durch ein Steuergerät an das Auslassventil geschickt wird. Neben dem Schließen des Auslassventils mittels eines Steuersignals kann auch das Öffnen des Auslassventils durch ein derartiges Steuersignal erfolgen. Dies ermöglicht es, den Medienaustrag im

Öffnungszeitraum Δt vollständig zeitlich von der Druckbeaufschlagung des Mediums in der Druckkammer zu entkoppeln. So kann der Medienaustrag in Reaktion auf das Betätigen eines Tasters am Spender beginnen und enden, sobald die gewünschte Menge des Mediums ausgetragen wurde.

Besonders vorteilhaft ist es, wenn im zweiten Schritt das Auslassventil bei Erreichen eines vorgegebenen Öffnungsdrucks p2 des Mediums geöffnet wird, der geringer als der Druck p1 ist. Vorzugsweise geschieht dies unmittelbar durch den Flüssigkeitsdruck des Mediums. Durch eine solche Ausgestaltung kann das Öffnen des Auslassventils bei einem definierten Druck erfolgen, ohne dass hierfür ein Drucksensor erforderlich ist. Stattdessen wird das Auslassventil durch das Medium selbst in Richtung seiner Öffnungsstellung gedrückt, sobald durch Druckbeaufschlagung des Mediums der Öffnungsdruck p2 erreicht wurde. Bei einer Ausgestaltung hiervon wird der beginnende Öffnungsvorgang des Auslassventils durch eine entsprechende Sensorik wahrgenommen und in Reaktion durch ein elektrisches Steuersignal der Öffnungsvorgang des Auslassventils unterstützt. Das Öffnen des Auslassventils erfolgt vorzugsweise gegen eine Federkraft und/oder gegen eine elektrisch bewirkte Schließkraft, durch die der Druck p2 vorgegeben wird.

Besonders vorteilhaft ist es, wenn das Auslassventil bei Erreichen des Öffnungsdrucks p2 in einer dem Auslassventil zugeordneten Ventilkammer geöffnet wird, wobei die Ventilkammer durch einen schmalen Kanal von der Druckkammer getrennt ist. Bei einer solchen Ausgestaltung sind beidseitig des schmalen Kanals beim Öffnen des Auslassventils die jeweiligen Drücke p1 und p2 bekannt, so dass der Volumenstrom durch den Kanal, der dem durch das Auslassventil ausgetragenen Volumenstrom entspricht, genau berechnet werden kann.

Besonders bevorzugt ist eine Ausgestaltung des Verfahrens, bei dem der zweite und der dritte Schritt des Austragvorgangs wiederholt werden, bis ein gewünschtes Austragvolumen und/oder eine gewünschte Anzahl an Zyklen erreicht ist, wobei die Wiederholung vorzugsweise mit einer Frequenz > 20 Hz, insbesondere größer > 100 Hz oder sogar größer > 1000 Hz, erfolgt. Durch das schnelle Schließen und Öffnen des Auslassventils ergibt sich eine Austragcharakteristik mit kleinen Tröpfchen. Dies ist insbesondere bei Spendern für ophtalmische Anwendung von Vorteil, da die kleinen Tröpfchen bei ihrem Auftreffen auf dem Auge eines Benutzers keinen Blinzelreflex hervorrufen. Während das Schließen des Auslassventils bei einer solchen Ausgestaltung durch ein elektrisches Steuersignal bewirkt wird, kann das jeweilige Öffnen entweder auch durch ein elektrisches Steuersignal oder alleine durch den Flüssigkeitsdruck p2 des Mediums in der Ventilkammer erfolgen.

Bei einer Weiterbildung der Erfindung geht dem Austragvorgang ein Messvorgang zur Erfassung von auf einen Ventilkörper des Austragventils und/oder auf einen Kolben der Druckerzeugungsvorrichtung wirkenden Kräften voraus. Zur Bewegung des Ventilkörpers bzw. des Kolbens sind vorzugsweise eine elektrische Ventilbetätigungseinrichtung und/oder eine elektrische Pumpenbetätigungseinrichtung vorgesehen. Durch den Messvorgang vor dem Austragvorgang kann ermittelt werden, welche über die von den Betätigungseinrichtungen bewirkten Kräfte hinausgehenden Nebenkräfte wie Federkräfte oder Reibungskräfte auf den Ventilkörper bzw. den Kolben wirken und dadurch Einfluss auf die Bewegung des Ventilkörpers oder des Kolbens während des Austragvorgangs nehmen. Die so erfassten Werte erlauben eine genauere Dosierung durch einen entsprechend angepassten Öffnungszeitraum oder durch Kompensieren der erfassten Kräfte. Der Messvorgang erfolgt vorzugsweise in einem Zustand, in dem in der Ventilkammer und/oder der

Der Messvorgang erfolgt unter Nutzung einer Ventilmesseinrichtung bzw. einer Pumpenmesseinrichtung. Diese gestatten es, die Bewegungsdauer und/oder die Verlagerung des Ventilkörpers bzw. des Pumpenkolbens zu erfassen. Unter Hinzunahme weiterer bekannter Parameter wie beispielsweise der Verlagerungsstrecke oder der durch die jeweilige Betätigungseinrichtung abgegebenen Leistung kann ermittelt werden, welche Nebenkräfte in welcher Höhe und Richtung die Bewegung des Ventilkörpers bzw. des Kolbens beeinflussen.

Da insbesondere Reibungskräfte am Ventilkörper oder am Pumpenkolben anfänglich deutlich höher sind als nach einigen Hüben, werden vorzugsweise vor Durchführung des Messvorgang mittels der Ventilbetätigungseinrichtung bzw. der Pumpenbetätigungseinrichtung mehrere Hübe des Ventilkörpers oder des Pumpenkolbens durchlaufen, um die Reibung zu verringern.

Der Messvorgang oder die Messvorgänge werden vorzugsweise nur einmalig bei der ersten Inbetriebnahme des Spenders durchgeführt und in einem vorzugsweise nicht-flüchtigen Speicher hinterlegt.

Die Öffnungszeit Δt kann abhängig vom Verlauf eines Austragvorgangs variabel sein. Vorzugsweise ist die Öffnungszeit Δt sowie vorzugsweise auch die Anzahl der Zyklen jedoch vor Beginn des Austragsvorgangs festgelegt.

Die Öffnungszeit Δt kann dabei bereits ab Werk festgelegt sein, wobei der Austrag eines vorgegebenen Volumens dadurch erzielt wird, dass in oben beschriebener Art und Weise ein Durchflussbegrenzer verwendet wird, der bei Einhaltung einer vorgegebenen beidseitigen Druckdifferenz einen definierten Volumenstrom erzeugt. Die Öffnungszeit Δt kann jedoch auch spenderspezifisch oder auch anwendungsspezifisch festgelegt werden. In einem solchen Fall ist es nicht zwingend erforderlich, dass eine vorgegebene Druckdifferenz zwischen den beiden Seiten des Durchflussbegrenzers eingehalten wird. Stattdessen kann die tatsächliche Druckdifferenz erfasst bzw. anhand erfasster Einflussgrößen berechnet werden und abhängig davon die Öffnungszeit Δt festgelegt werden.

Bei beiden Varianten können demnach vor Beginn des Austragsvorgangs die auf den Ventilkörper oder den Kolben wirkenden spenderspezifischen Nebenkräfte erfasst werden, wobei die erfassten Nebenkräfte bei der ersten Variante genutzt werden, um die vorgegebenen Drücke p1 und p2 mittels Ausgleichsmitteln in Form einer Ventilbetätigungseinrichtung und/oder eine Pumpenbetätigungsvorrichtung zu erzeugen. Bei der zweiten Variante werden die erfassten Nebenkräfte herangezogen, um die austragsvorrichtungsspezifischen Drücke p1 und p2 zu berechnen und Δt in Abhängigkeit dieser Drücke für die Austragsvorrichtung und das auszutragende Volumen spezifisch festzulegen.

Bei einer Weiterbildung des Verfahrens geht dem Austragvorgang ein Erstbefüllungsvorgang voraus, bei dem nach dem Öffnen des Auslassventils durch ein elektrisches Steuersignal vorhandene Luft aus dem Strömungspfad zwischen der Druckkammer und einer Auslassöffnung mittels der Druckerzeugungseinrichtung verdrängt wird. Die Luftverdrängung ist erforderlich, um beim Austragvorgang eine definierte Medienmenge austragen zu können. Durch die mittels des elektrischen Steuersignals bewirkte Öffnung des Auslassventils kann die Luft in sehr einfacher Art und Weise entfernt werden, indem die Druckerzeugungseinrichtung das Volumen des Strömungspfads reduziert und dabei die Luft durch die Auslassöffnung hindurch nach außen drückt. Dieser Vorgang kann mehrfach wiederholt werden, wobei beispielsweise durch Schließen des Auslassventils oder durch Schließen eines zusätzlichen Rückschlagventils während der Vergrößerung des Druckkammervolumens verhindert wird, dass Luft durch die Auslassöffnung wieder in den Strömungspfad eintritt.

Die Erfindung betrifft weiterhin ein Herstellungsverfahren zur Herstellung eines Spenders zum Austrag eines flüssigen pharmazeutischen Mediums in oder auf dem Körper eines Benutzers, bei der spezifische Parameter des Spenders im Zuge der Herstellung gemessen oder ermittelt werden und in einem vorzugsweise nicht-flüchtigen Speicher des Spenders gespeichert werden. Bei den spezifischen Parametern handelt es sich um Parameter, die auch bei identischer Herstellungsmethode von Spender zu Spender oder Herstellungscharge zur Herstellungscharge variieren können. Diese spezifischen Parameter werden während der Herstellung ermittelt und anschließend in dem Speicher des Spenders gespeichert, um bei einem Austragvorgang mit einbezogen zu werden. Die spezifischen Parameter können beispielsweise Reibungskräfte an einem Ventilkörper oder einem Kolben, Federkonstanten einer Federeinrichtung oder verschiedene Maße des Spenders wie beispielsweise Zylinderdurchmesser umfassen. Vorzugsweise werden diese Werte individuell für jeden Spender ermittelt. Es ist jedoch auch möglich, die Werte jeweils nur für ganze Chargen, beispielsweise jeweils 100 oder 1.000 Spender, zu ermitteln, um bei Parametern, die erfahrungsgemäß je Charge etwa gleich bleiben, den Aufwand zur Ermittlung der spezifischen Parameter gering zu halten.

Die gespeicherten Parameter können Anwendung finden, um die Abweichung von einem gewünschten Austragvolumen möglichst klein zu halten, indem die Wirkung der Abweichung in Hinblick auf das ausgetragene Volumen jeweils durch andere Maßnahmen kompensiert wird. So kann beispielsweise ein zu großer Durchmesser des Verbindungskanals des Durchflussbegrenzers zwischen Druckkammer und Ventilkammer durch die Herstellung einer geringeren Druckdifferenz zwischen Druckkammer und Ventilkammer kompensiert werden.

### Kurzbeschreibung der Zeichnungen

Weitere Aspekte und Vorteile der Erfindung ergeben sich außer aus den Ansprüchen auch aus der nachfolgenden Beschreibung von drei bevorzugten Ausführungsformen der Erfindung, die anhand der Figuren beschrieben sind. Dabei zeigen:
- Fig. 1: eine erste Ausführungsform eines erfindungsgemäßen Spenders mit elektrischer Ventilbetätigungseinrichtung und elektrischer Pumpenbetätigungseinrichtung sowie mit Durchflussbegrenzer,
- Fig. 2: eine zweite Ausführungsform eines erfindungsgemäßen Spenders mit elektrischer Ventilbetätigungseinrichtung und manueller Pumpenbetätigungseinrichtung sowie mit Durchflussbegrenzer,
- Fig. 3: eine dritte Ausführungsform eines erfindungsgemäßen Spenders mit elektrischer Ventilbetätigungseinrichtung und manueller Pumpenbetätigungseinrichtung ohne Durchflussbegrenzer und
- Fig. 3a: eine Variante der dritten Ausführungsform eines erfindungsgemäßen Spenders mit Durchflusssensor.

### Detaillierte Beschreibung der Ausführungsbeispiele

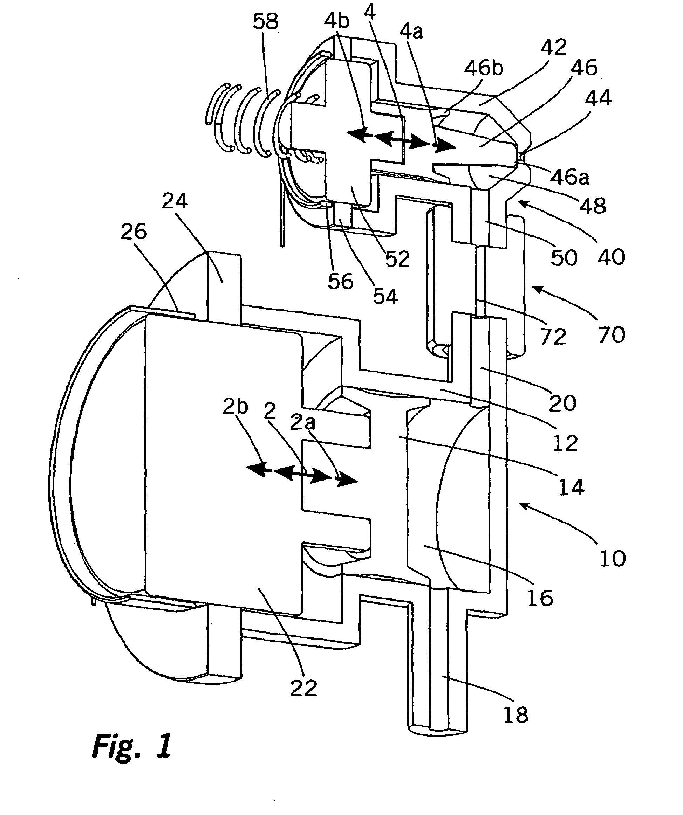

Fig. 1 zeigt eine erste Ausführungsform eines erfindungsgemäßen Spenders. Dabei sind vom Spender nur jene unmittelbar für den Austragvorgang vorgesehenen Bauteile dargestellt. Nicht dargestellt sind ein den Spender umgebendes Gehäuse, das Medienreservoir sowie ein Steuergerät zur Steuerung des Austragsvorgangs sowie mit dem Steuergerät verbundene Betätigungstaster sowie eine Anzeigevorrichtung.

Die in Fig. 1 dargestellten Komponenten umfassen eine Druckerzeugungseinrichtung 10, eine Auslassventileinheit 40 sowie einen Durchflussbegrenzer 70, der die Druckerzeugungseinrichtung 10 mit der Auslassventileinheit 40 verbindet.

Die Druckerzeugungseinrichtung 10 weist einen Zylinder 12 auf, innerhalb dessen ein Kolben 14 entlang der Bewegungsrichtung 2 beweglich ist. Der Zylinder 12 und der Kolben 14 begrenzen gemeinsam eine durch die Bewegung des Kolbens 14 volumenveränderliche Druckkammer 16. Die Druckkammer 16 ist über einen Einlasskanal 18 mit dem nicht dargestellten Reservoir verbunden, in dem das Medium gespeichert ist. Über einen Auslasskanal 20 ist der Zylinder 12 mit dem Durchflussbegrenzer 70 verbunden. Der Einlasskanal 18 und der Auslasskanal 20 münden bezogen auf die Bewegungsrichtung 2 des Kolbens 14 versetzt zueinander in die Druckkammer 16. Dies führt dazu, dass bei einer Bewegung des Kolbens in Richtung 2a zunächst die Druckkammer von dem Einlasskanal 18 getrennt wird, so dass das Medium nicht mehr zurück in das Reservoir gelangen kann. Erst anschließend kann durch das Weiterbewegen des Kolbens 14 in Richtung 2a eine Druckbeaufschlagung des in der Druckkammer 16 befindlichen Mediums erfolgen.

Der Kolben 14 ist fest verbunden mit einem permanentmagnetischen Kern 22, der auf der der Druckkammer 16 abgewandten Seite des Kolbens 14 auf diesen aufgesteckt ist. Der Kern 22 ist innerhalb zweier den Kern 22 umgebenden Spulen 24, 26 angeordnet, wobei die Spule 24 eine Pumpenbetätigungsspule 24 und die Spule 26 eine Pumpenmessspule 26 darstellt.

Ein ähnlicher Aufbau wie bei der Druckerzeugungseinrichtung 10 findet sich auch bei der Auslassventileinheit 40. Diese weist ein abschnittweise zylindrisches Ventilgehäuse 42 auf, an dessen Stirnende eine Auslassöffnung 44 vorgesehen ist. Innerhalb des Ventilgehäuses 42 ist ein in Richtung 4 verschiebbarerer Ventilkörper 46 vorgesehen, der in seiner Endlage in Richtung 4a die Auslassöffnung 44 mit einem Schließabschnitt 46a verschließt. Auf der gegenüberliegenden Seite des Ventilkörpers 46 weist dieser eine nach außen weisende umlaufende Lippe 46b auf, die an der Innenseite des Ventilgehäuses 42 anliegt. Gemeinsam begrenzen der Ventilkörper 46 und das Ventilgehäuse 42 eine Ventilkammer 48. Über einen Einlasskanal 50 ist die Ventilkammer 48 mit dem Durchflussbegrenzer 70 verbunden.

Wie auch der Kolben 14 der Druckerzeugungsvorrichtung 10 ist der Ventilkörper 46 auf seiner rückwärtigen Seite mit einem Kern 52 fest verbunden, wobei der Kern 52 von einer Ventilbetätigungsspule 54 sowie einer Ventilmessspule 56 umgeben ist. Abweichend von der Ausgestaltung der Druckerzeugungsvorrichtung 10 weist die Ventileinheit 40 zusätzlich eine Schraubenfeder 58 auf, mittels derer der Kern 52 sowie der Ventilkörper 46 permanent in Richtung 4a kraftbeaufschlagt sind.

Der Auslasskanal 20 der Druckerzeugungsvorrichtung 10 und der Einlasskanal 50 der Ventileinheit 40 sind mittelbar über den Durchflussbegrenzer 70 miteinander verbunden. Dieser weist einen schmalen Verbindungskanal 72 auf, durch den das Medium aus der Druckkammer 16 in die Ventilkammer 48 gelangen kann.

Der dargestellte und beschriebene Aufbau ermöglicht es, mittels der Druckerzeugungsvorrichtung 10 das Medium, welches durch den Einlasskanal 18 in die Druckkammer 16 gefördert wurde, durch eine Kraftbeaufschlagung des Kolbens 14 in Richtung 2a mit einem Druck zu beaufschlagen. Hierdurch erhöht sich der Druck des Mediums auch in der Ventilkammer 48. Nach Öffnen des Auslassventils 40 durch Verlagerung des Ventilkörpers 46 in Richtung 4b kann das unter Druck stehende Medium durch die Auslassöffnung 44 ausgetragen werden.

Die Besonderheit des dargestellten Spenders liegt insbesondere in der Gestaltung der Ventileinheit 40. Wie die aus dem Stand der Technik bekannten Auslassventile weist auch die Ventileinheit 40 mit dem Ventilkörper 46 einen Ventilkörper auf, der gegen die Federkraft der Feder 58 in Folge eines Druckanstiegs in der Ventilkammer 48 verlagert werden kann und so die Auslassöffnung 44 freigibt. Neben der Kraftbeaufschlagung des Ventilkörpers 46 durch die Feder 58 einerseits und den Flüssigkeitsdruck in der Ventilkammer 48 andererseits ist eine Kraftbeaufschlagung des Ventilkörpers 46 jedoch auch mittels der Ventilbetätigungsspule 54 möglich. Wenn diese Spule 54 von einem Strom durchflossen wird, so führt dies zur Ausbildung eines Magnetfeldes, durch welches der Kern 52 in Abhängigkeit der Stromflussrichtung und der Stromstärke in der Spule 54 mit einer variablen Kraft in Richtung 4a oder 4b kraftbeaufschlagt wird. Es ist demnach durch die Ventilbetätigungsspule 54 möglich, ohne das Vorliegen eines Flüssigkeitsdrucks in der Ventilkammer 48 den Ventilkörper in Richtung 4b zu verlagern und/oder trotz des Vorliegens eines zum Überwinden der Federkraft der Feder 58 ausreichend hohen Flüssigkeitsdrucks in der Ventilkammer 48 den Ventilkörper 46 in Richtung 4a zu verlagern, um das Ventil somit zu schließen oder geschlossen zu halten.

Neben der Ventilbetätigungsspule 54 ist die Ventilmessspule 56 vorgesehen, mittels derer die Verlagerung des Kerns 52 erfasst werden kann. Eine Bewegung des Kerns 52 führt dazu, dass an den Anschlüssen der Ventilmessspule 56 eine Spannung anliegt, die zum Zwecke der Auswertung erfasst werden kann. Dabei ist es sowohl möglich, über die Ventilmessspule 56 zu erkennen, ob der Kern 52 bewegt wird, als auch, wie schnell der Kern 52 bewegt wird.

Wie auch die Ventileinheit 40 weist die Druckerzeugungseinrichtung ebenfalls eine Pumpenbetätigungsspule 24 sowie eine Pumpenmessspule 26 auf. Die Pumpenbetätigungsspule 24 gestattet es, den Kern 22 gemeinsam mit dem Kolben 14 in Richtung 2a und anschließend in entgegengesetzte Richtung 2b verlagern. Bei der Verlagerung in Richtung 2a wird der Zugang 18 der Druckkammer 16 geschlossen und anschließend das Medium in der Druckkammer 16 druckbeaufschlagt, bis es nach Öffnen des Auslassventils 40, verursacht durch den Druck in der Ventilkammer, zu einem Austragvorgang kommt. Bei der nachfolgenden Verlagerung des Kolbens 14 in Richtung 2b entsteht ein Unterdruck in der Druckkammer 16, der zu einem Ansaugen neuen Mediums aus dem Reservoir führt, sobald der Zugang 18 vom Kolben 14 wieder freigegeben wird.

Wie auch bei der Ventileinheit 40 und dem Ventilkörper 46 wird die Bewegung des Kolbens dadurch erreicht, dass mittels der Pumpenbetätigungsspule 24 ein Magnetfeld aufgebaut wird, durch das der permanentmagnetische Kern 22 kraftbeaufschlagt wird. Anders als beim Ventil 40 ist jedoch keine zusätzliche Feder vorgesehen. Die Pumpenbetätigungsspule 24 stellt somit die einzige Möglichkeit dar, den Kolben 14 zum Zwecke der Druckbeaufschlagung des Mediums in der Kammer 16 in Richtung 2a zu verlagern. Die Pumpenmessspule 26 erfasst die Bewegung des Kerns 22 und erlaubt dadurch eine Erkennung, ob der Kern 22 sich bewegt und/oder wie schnell der Kern 22 sich bewegt.

Vor dem Hintergrund dieses Aufbaus läuft der Austragvorgang folgendermaßen ab:
Sobald das Medium in der Druckkammer 16 durch den Kolben 14 kraftbeaufschlagt wird, so dass sich dort ein Flüssigkeitsdruck p1 einstellt, erhöht sich verzögert auch der Druck in der Ventilkammer 48, bis dort der geringere Druck p2 vorliegt. Dieser Druck p2 führt dazu, dass der Ventilkörper 46 in Richtung 4b entgegen der Federkraft der Feder 58 verlagert wird und somit die Auslassöffnung 44 freigibt. Das in der Ventilkammer 48 befindliche Medium kann dadurch in Form eines Strahls an die Umgebung austreten. Der Zeitpunkt, zu dem im Zuge des Öffnens die Verlagerung des Ventilkörpers 46 stattfindet, wird von dem nicht dargestellten Steuergerät mittels der Ventilmessspule 56, die auf die Bewegung des Kerns 52 mit einem an ihren Kontakten anliegenden Spannung reagiert, erfasst. Gegebenenfalls muss das Steuergerät, welches die Spannung auswertet, dabei jene Spannungsinduzierung reduzierend berücksichtigen, die durch die Bestromung der Ventilbetätigungsspule 54 und unabhängig von der Bewegung des Kerns 52 erzielt wird.
Sobald die gewünschte Medienmenge nach einer Zeitspanne Δt ausgetragen wurde, wird das Auslassventil 40 geschlossen, indem durch die Ventilbetätigungsspule 54 der Ventilkörper mit einer in Richtung 4a wirkenden Kraft beaufschlagt wird, durch die der Ventilkörper 46 gegen den Flüssigkeitsdruck p2 an die Ventilöffnung 44 angepresst wird. Der Austragvorgang ist damit beendet.

Nach Beendigung des Austragvorgangs kann der Kolben 14 wieder in seine in Fig. 1 dargestellte Lage zurückgefahren werden, so dass sich ein Unterdruck in der Druckkammer 16 bildet, durch den Medium aus dem Reservoir nachgesogen wird.

Der Volumenstrom, der durch die Auslassöffnung 44 ausgetragen wird, wird maßgeblich durch die Geometrie des Durchflussbegrenzers 70 als Teil des Strömungspfades mit dem größten Strömungswiderstand und durch die Drücke p1 und p2 beidseitig des Durchflussbegrenzers 70 bestimmt. Die Drücke p1 und p2 bleiben über den Austragvorgang hinweg weitgehend konstant bleiben, da die durch die Pumpenbetätigungsspule 24 aufgebrachte Kraft und damit der Druck p1 in der Druckkammer unverändert bleibt und da das Auslassventil 40 für einen konstanten Druck in der Ventilkammer 48 sorgt. Aufgrund konstanter Drücke p1, p2 bleibt auch der Volumenstrom durch den Durchflussbegrenzer, also das pro Zeiteinheit durch den Durchflussbegrenzer strömende Volumen, über den Austragvorgang unverändert.

Da der Volumenstrom konstant ist, bestimmt alleine die Öffnungszeit des Auslassventils 40 die Menge des ausgetragenen Mediums. Diese Proportionalität ermöglicht es, das gewünschte Austragsvolumen dadurch zu erreichen, dass nach einer einfach berechenbaren oder vorgegebenen Zeitspanne das Ventil 40 wieder geschlossen wird.

Um mit dem dargestellten Spender ein vordefiniertes Volumen austragen zu können, sind mehrere Vorgehensvarianten möglich.

Bei einer ersten Variante ist die Öffnungszeit Δt des Auslassventils zur Abgabe des Volumens V fest vorgegeben. Um innerhalb dieser Zeitspanne Δt dieses Volumen V auszugeben, ist die Erzielung eines definierten Volumenstroms im Durchflussbegrenzer erforderlich. Dieser kann erreicht werden, wenn die Drücke p1 und p2 oder zumindest die Druckdifferenz Δp = p1 - p2 einen vorgegebenen Wert einnimmt.

Der Druck p1 hängt maßgeblich von der Kraftbeaufschlagung des Kolbens 14 mittels der Pumpenbetätigungsspule 24 und dem Kern 22 ab. Allerdings kann insbesondere aufgrund von Reibungskräften zwischen dem Kolben 14 und dem Zylinder 12 eine Kraftbeaufschlagung des Kolbens mittels der Pumpenbetätigungsspule 24 zu einem abweichenden Druck in der Druckkammer 16 führen.

Um die zur Erreichung des Drucks p1 erforderliche Kraftbeaufschlagung durch die Pumpenbetätigungsspule 24 zu ermöglichen, werden gemäß dieser ersten Variante daher zunächst die Reibungskräfte zwischen dem Kolben 14 und dem Zylinder 12 und/oder andere zu Ungenauigkeiten führende Nebenkräfte erfasst. Hierzu wird in einem Zustand, in dem die Druckkammer 16 noch luftbefüllt ist, der Kolben 14 aus seiner einen Endlage in seine andere Endlage überführt und die hierfür benötigte Zeit erfasst. Diese ist umso größer, je größer die zwischen Kolben 14 und Zylinder 12 wirkenden Reibungskräfte sind. Dieser Vorgang wird zum Ausschließen von Messfehlern vorzugsweise mehrfach wiederholt. Die dadurch erfassten Reibungskräfte werden herangezogen, um die erforderliche Kraftbeaufschlagung des Kolbens 14 mittels der Pumpenbetätigungsspule 24 zu errechnen, die zu einer Druckbeaufschlagung des Mediums in der Druckkammer mit dem Druck p1 führt.

In gleicher Art und Weise kann auch die Berechnung einer Korrekturkraft erfolgen, mit der die Ventilbetätigungsspule 54 den Ventilkörper 46 beaufschlagen muss, damit das Auslassventil 40 bei einem Druck in der Höhe des vorgegebenen Wertes p2 öffnet. Obwohl der Druck, bei dem das Auslassventil 40 öffnet, maßgeblich von der Auslegung der Feder 58 abhängt, kann der hierdurch bewirkte Öffnungsdruck aufgrund von Abweichungen bei den Parametern der Feder sowie aufgrund von Reibungskräften zwischen dem Ventilkörper 46 und dem Ventilgehäuse 42 streuen. Dies kann durch eine zusätzliche Kraftbeaufschlagung des Ventilkörpers 46 mittels der Pumpenbetätigungsspule 54 mit einer Korrekturkraft ausgeglichen werden.

Um beurteilen zu können, wie groß diese Korrekturkraft sein muss, wird im Zuge der Inbetriebnahme des Spenders mittels der Ventilbetätigungsspule und der Ventilmessspule eine Analyse des Ventils 40 durchgeführt werden. Hierzu wird der Ventilkörper 46 aus seiner Schließlage mittels einer definierten Leistung der Betätigungsspule 54 in seine Öffnungsstellung überführt. Die hierfür benötigte Zeit wird erfasst. Sie ist umso größer, je größer die Nebenkräfte sind, die einer Verlagerung entgegenwirken. Aus den Ergebnissen der Analyse lässt sich eine Korrekturkraft berechnen, die bei einer gleichzeitigen Kraftbeaufschlagung des Ventilkörpers mit der Federkraft und dieser Korrekturkraft gewährleistet, dass das Auslassventil sich bei dem vorgegebenen Druck p2 öffnet.

Da somit sowohl die vorgegebenen Drücke p1 als p2 exakt eingehalten werden, wird während des Austrags des Mediums auch der vorgegebene Volumenstrom durch den Durchflussbegrenzer erzielt, so dass in der vorgegebenen Zeitspanne Δt die vorgegebene Menge des Mediums ausgetragen wird.

Beispielhaft seien folgende Werte genannt: Vorgegeben ist, dass ein Volumen von 50 µl in einem Öffnungszeitraum von 7 * 10⁻² sec ausgetragen werden soll. Um dies bei einer Dimensionierung des Verbindungskanals 72 mit einem Durchmesser von D = 0,25 mm, einer Länge von l = 4 mm und einer beispielhaft angenommenen Viskosität von µ = 1 mPa s (Wasser) zu erreichen, muss gemäß der Formel q = Δp · (π · D⁴ ) / (128 · µ · l) eine Druckdifferenz Δp von 0,3 bar vorliegen. Diese Druckdifferenz soll dadurch erzielt werden, dass p1 = 4,3 bar und p2 = 4 bar vorgegeben ist.

Um diese Werte p1 und p2 zu erreichen, werden in oben beschriebener Art und Weise die Reibungskräfte und die Abweichung an der Federkraft bestimmt, um mittels der Ventilbetätigungsspule 54 und der Pumpenbetätigungsspule 24 jeweils eine Kraftbeaufschlagung des Kolbens 14 und des Ventilkörpers 46 zu erzielen, die diese Drücke p1 und p2 zur Folge haben. Beispielsweise kann im Falle einer zu geringen Federkraft der Feder 58 durch eine zusätzliche von der Ventilbetätigungsspule 54 erzeugte Korrekturkraft in Richtung 4a kompensiert werden.

Bei einer zweiten Variante ist das auszutragende Volumens V fest vorgegeben, die Öffnungszeit Δt des Auslassventils jedoch nicht. Statt eine Anpassung der Kraftbeaufschlagung durch die Ventilbetätigungsspule 54 und der Pumpenbetätigungsspule 24 vorzunehmen, um vorgegebene Drücke p1 und p2 zu erzeugen, werden durch die Messvorgänge mittels der Pumpenmessspule 26 und der Ventilmessspule 56 lediglich die sich tatsächlich einstellenden Drücke p1 und p2 errechnet. Diese können von Spender zu Spender variieren.

Nach Ermittlung der Drücke p1 und p2 durch Erfassung der auf den Ventilkörper 46 und den Kolben 14 wirkenden Nebenkräfte, wird vom Spender selbst die Öffnungszeit Δt berechnet, die erforderlich ist, um das vorgegebene Volumen auszutragen.

Beispielhaft seien folgende Werte genannt: Vorgegeben ist, dass ein Volumen von 50 µl ausgetragen werden soll. Ermittelt wird im Rahmen des Messvorgangs, dass der Druck p1 = 4,2 bar und der Druck p2 = 4,0 bar beträgt. Daraus ergibt sich unter Berücksichtigung der Dimensionierung des Verbindungskanals 72 mit einem Durchmesser von D = 0,25 mm und einer Länge von l = 4 mm, dass die Durchflussrate q durch den Verbindungskanal 72 nach oben genannter Formel 479 · 10⁻⁶ l / sec beträgt. Der Spender legt anhand dessen fest, dass zum Austrag von 50 µl eine Öffnungszeit Δt = 1,04 · 10⁻¹ l sec erforderlich ist.

Durch beide Varianten ist es demnach möglich, trotz spezifischen Abweichungen des Spenders ein vorgegebenes Volumen des Mediums zuverlässig auszutragen. Unabhängig vom Betriebsmodus gestattet der erläuterte Spender demnach auf Basis bekannter Drücke p1 und p2 sowie auf Basis der definierten Geometrie des Durchflussbegrenzers 70 und der Eigenschaften des Mediums die Erzielung eines Austragvorgangs mit konstantem Volumenstrom. Das wiederum ermöglicht ein genau einstellbares Austragvolumen alleine über die Steuerung der Öffnungszeit des Auslassventils 40.

Alternativ zum beschriebenen Austragvorgang, bei dem das gewünschte Volumen kontinuierlich ausgetragen wird, kann auch ein gepulster Austragvorgang vorgesehen sein, bei dem jeweils nach festgelegter Öffnungsintervalldauer von beispielsweise 0,5 Millisekunden das Auslassventil kurz mittels der Ventilbetätigungsspule 54 geschlossen wird. Unmittelbar im Anschluss oder nach einer festgelegten Zeitdauer von beispielsweise ebenfalls 0,5 Millisekunden wird die Kraftbeaufschlagung durch die Ventilbetätigungsspule 54 wieder entfernt, so dass das Auslassventil durch den Flüssigkeitsdruck in der Ventilkammer 48 wieder öffnet. Aufgrund der festgelegten Öffnungsintervalldauer wird dabei je Öffnungsvorgang eine festgelegte Menge des Mediums ausgetragen, so dass durch die Anzahl der Öffnungsvorgänge das ausgetragene Gesamtvolumen festgelegt werden kann. Der beschriebene gepulste Austrag des Mediums ist vor allem bei ophtalmischer Verwendung zweckmäßig, da er es gestattet, sehr kleine Tröpfchengrößen zu erzeugen, die kein Blinzeln des Benutzers verursachen.

Ein besonderer Vorteil, den die Ausführungsform der Fig. 1 bietet, ist die einfache Möglichkeit, im Lieferzustand im Flüssigkeitspfad zwischen dem Reservoir und der Auslassöffnung 44 befindliche Luft aus dem Strömungspfad zu verdrängen. Hierzu wird bei der Erstinbetriebnahme mittels der Ventilbetätigungsspule 54 ein Öffnen des Auslassventils 40 bewirkt, so dass ein anschließend durch die Druckerzeugungsvorrichtung 10 aufgebrachter Druck ein Hinausströmen der Luft durch die Auslassöffnung 44 bewirkt. Nachdem der Kolben 14 dabei seine Hubendlage in Richtung 2a erreicht hat, wird das Auslassventil 40 geschlossen und der Kolben in Richtung 2b zurückgefahren, bis aufgrund des sich einstellenden Unterdrucks durch den Einlasskanal 18 Medium aus dem Reservoir in die Druckkammer 16 strömt. Dieser Vorgang wird mehrfach wiederholt, so dass schrittweise die gesamte Luft aus dem Strömungspfad verdrängt und durch Medium ersetzt wird. Der Abschluss dieses Vorgangs kann mittels der Pumpenmessspule 26 erfasst werden, da die Verlagerung des Kolbens in Richtung 2a gegen einen zunehmenden Widerstand erfolgt. So steigt die zur Verlagerung des Kolbens 14 erforderliche Zeit signifikant an, sobald das bis zu diesem Zeitpunkt aus dem Reservoir geförderte Medium im Zuge der Verlagerung des Kolbens den Kanal 72 durchqueren muss. Auf Basis dieser Information kann das Steuergerät abschätzen, inwieweit die Luft aus dem Strömungspfad bereits verdrängt wurde.

Zur Überprüfung der Verdrängung der Luft kann folgendes Verfahren angewandt werden. Zunächst wird mittels der Ventilsteuerspule 54 das Auslassventil 40 geschlossen, wobei der Ventilkörper 46 mit einer so großen Kraft beaufschlagt wird, dass selbst bei einem Flüssigkeitsdruck p1 in der Ventilkammer 48 das Ventil nicht öffnet. Anschließend wird der Kolben 14 aus seiner Ausgangslage der Fig. 1 in Richtung 2a verlagert. Sofern bereits die gesamte Luft aus dem Strömungspfad verdrängt wurde, ist eine Verlagerung des Kolbens aufgrund der Inkompressibilität der Flüssigkeit nur bis zu der Zwischenlage möglich, in der der Kolben gerade den Einlasskanal 18 passiert hat. Wenn jedoch der Kolben über diese Lage hinaus in Richtung 2a bewegbar ist, so ist dies ein sicheres Zeichen dafür, dass noch Luft im Strömungspfad verblieben ist. In einem solchen Fall kann durch das Steuergerät das Auslassventil 40 geöffnet werden, um diese Luft entweichen zu lassen.

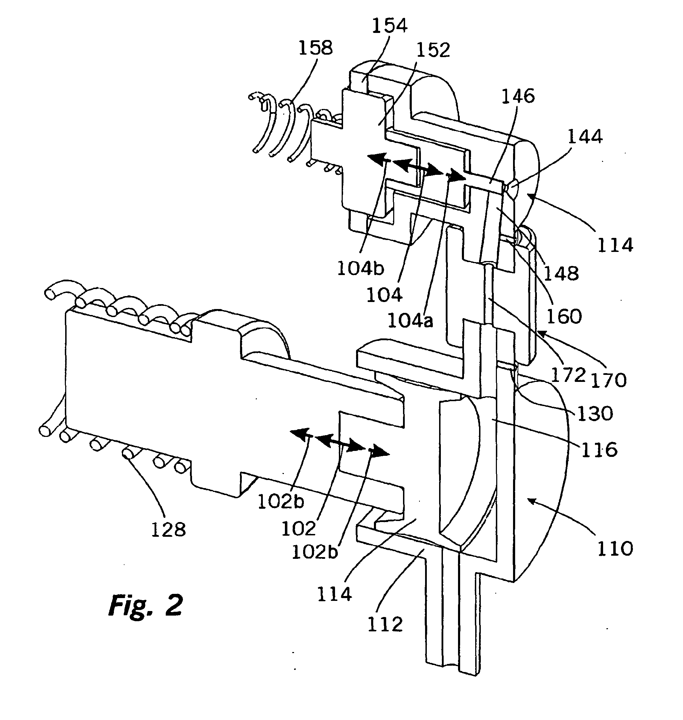

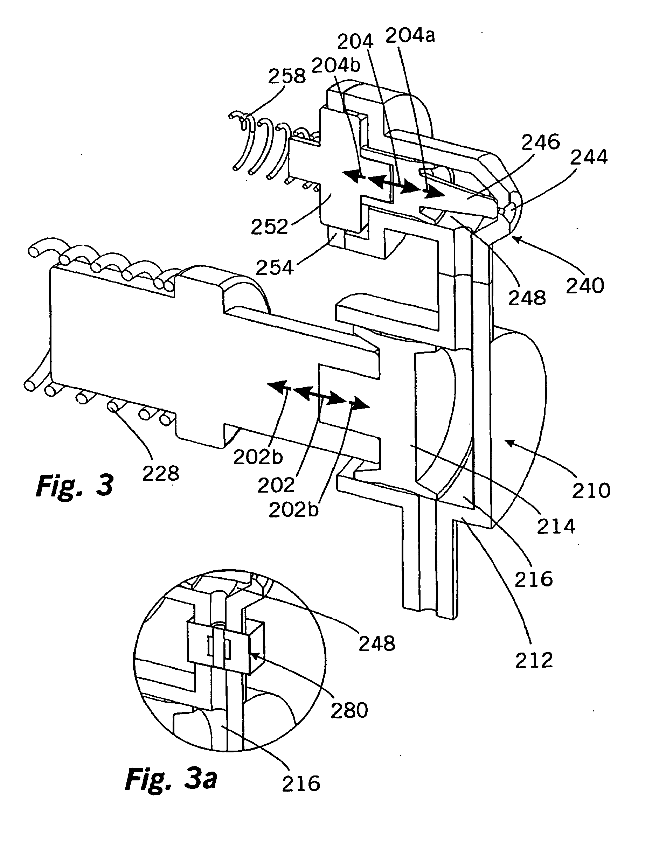

Die Ausführungsformen der Fig. 2 und 3 unterscheiden sich von der Ausführungsform der Fig. 1 bezüglich einiger Aspekte. Hinsichtlich der nicht als unterschiedlich gekennzeichneten Komponenten gelten die Ausführungen zu Fig. 1.

Die Druckerzeugungsvorrichtung 110 der Ausführungsform der Fig. 2 ist bei dieser Gestaltung dafür ausgebildet, den Druck mittels einer Feder 128 zu erzeugen, die durch einen nicht näher dargestellten Mechanismus zuvor durch den Benutzer durch Zurückziehen des Kolbens 114 manuell gespannt wird. Hinsichtlich der Ausgestaltung der Druckerzeugungsvorrichtung 110 als Kolbenpumpe mit Zylinder 112 und Kolben 114 stimmt die Ausführungsform mit der Ausführungsform der Fig. 1 überein. Allerdings sind weder eine Pumpenbetätigungsspule noch eine Pumpenmessspule vorgesehen. Hinsichtlich der Auslassventileinheit 140 liegt der maßgebliche Unterschied darin, dass das Auslassventil nicht mittels eines Flüssigkeitsdrucks auf mechanische Art und Weise in einen Öffnungszustand überführbar ist. Der Ventilkörper 146 weist keine Druckbeaufschlagungsfläche auf und ist daher lediglich mittels einer Ventilbetätigungsspule 154 entgegen der Federkraft der Feder 158 auslenkbar. Weiterhin ist neben Ventilbetätigungsspule 154 keine Ventilmessspule vorgesehen.

Als weiterer Unterschied ist vorgesehen, dass sowohl im Bereich der Pumpenkammer 116 als auch im Bereich der Ventilkammer 148 jeweils Drucksensoren 130, 160 vorgesehen sind. Mittels dieser Drucksensoren kann der Druck in den Kammern 116, 148 unmittelbar erfasst werden.

Die Funktionsweise der Ausführungsform der Fig. 2 sieht vor, dass die Druckbeaufschlagung des Mediums in der Druckkammer 116 durch die zuvor manuell gespannte Feder 128 erzeugt wird. Da die Federcharakteristik sowie die Reibungskräfte des Kolbens 114 an der Zylinderwandung 112 nicht exakt vorhersagbar sind, stellt sich in der Druckkammer 116 ein Druck p1 ein, der nicht exakt errechenbar ist, sondern lediglich mittels des Drucksensors 130 gemessen werden kann. Unter Zwischenschaltung des Durchflussbegrenzers 170 wirkt sich dieser Druck p1 auch auf das Medium im Ventilbereich 148 aus, dessen Druck ebenfalls steigt. Sobald ein festgelegter und gegenüber dem Druck p1 geringerer Druck p2 vom Drucksensor 160 gemessen wird, erfolgt durch eine Strombeaufschlagung der Ventilbetätigungsspule 154 eine Öffnung des Austragventils 140 durch Verlagerung des Ventilkörpers 146 in Richtung 104b. Der Ventilkörper wird dabei so weit verlagert, dass der Druck p2 in der Ventilkammer 148 aufrechterhalten bleibt. Während des resultierenden Austragvorgangs bleiben die Drücke p1 und p2 daher wiederum etwa konstant und durch die Drucksensoren 130, 160 erfassbar, was in der oben bereits beschriebenen Art und Weise gestattet, die Durchflussmenge durch den Durchflussbegrenzer 170 exakt zu berechnen. Selbst wenn sich der Druck p1 oder der Druck p2 während des Austragvorgangs ändern, ist dies mittels der Drucksensoren 130, 160 erfassbar, so dass das Steuergerät einen entsprechend verminderten oder erhöhten Volumenstrom zur Errechnung des bereits ausgetragenen Mediums heranzieht. Sobald die auszutragende Medienmenge erreicht ist, wird das Auslassventil 140 vom Steuergerät durch Kraftbeaufschlagung des Ventilkörpers 146 in Richtung 104a geschlossen.

Die Ausführungsform der Fig. 3 ähnelt der Ausführungsform der Fig. 2. Wiederum erfolgt die Kraftbeaufschlagung des Kolbens 214 mittels einer manuell spannbaren Feder 228. Abweichend von den vorausgegangenen Ausführungsformen ist jedoch kein Durchflussbegrenzer vorgesehen, so dass der Druck p1, der sich infolge der Federkraft der Feder 228 in der Druckkammer 216 einstellt, unmittelbar auch in der Ventilkammer 248 herrscht. Abweichend von der Ausgestaltung der Fig. 1 ist das Auslassventil 240 jedoch derart ausgebildet, dass aufgrund der Federkraft der Feder 258 des Auslassventils 240 der Öffnungsdruck p2, bei dem es alleine durch den Flüssigkeitsdruck des Mediums in der Ventilkammer 248 geöffnet wird, oberhalb des Drucks p1 liegt. Durch die Druckbeaufschlagung des Mediums mit dem Druck p1 wird also zunächst ein einheitlicher Flüssigkeitsdruck p1 in der Druckkammer 216 sowie der Ventilkammer 248 hergestellt, ohne dass es zu einem Austragvorgang kommt. Der Austragvorgang beginnt erst, sobald in Reaktion auf einen Tastendruck des Benutzers durch Strombeaufschlagung der Ventilbetätigungsspule 254 der Ventilkörper 246 in Richtung 204b verlagert wird. Der Austragvorgang wird nach einer vorgegebenen Öffnungszeit beendet, indem nach Wegfall der Strombeaufschlagung der Ventilbetätigungsspule 254 der Ventilkörper 246 wieder in Richtung 204a an die Auslassöffnung 244 angepresst wird.

Diese dritte Ausgestaltung gestattet es nicht, die ausgetragene Flüssigkeitsmenge genau zu bestimmen, da der Flüssigkeitsdruck p1 maßgeblich durch die Federkraft der Feder 228 bestimmt wird, deren spezifische Kennwerte von den Soll-Kennwerten abweichen können. Bei Medien, bei denen die exakte Dosierung weniger wichtig ist, kann die Ausführungsform der Fig. 3 jedoch verwendet werden.

Um eine genauere Dosierung zu erzielen, kann die Ausführungsform der Fig. 3 zusätzlich um eine Durchflussmesseinrichtung 280 ergänzt werden, die beispielsweise zwischen der Druckkammer 160 und der Ventilkammer 148 anzuordnen ist, wie in Fig. 3a schematisch dargestellt ist. Die Durchflussmesseinrichtung 280 erlaubt es, die ausgetragene Flüssigkeitsmenge direkt zu erfassen und durch Wegfall der Kraftbeaufschlagung des Ventilkörpers 246 in Richtung 204b oder durch Kraftbeaufschlagung des Ventilkörpers 246 in Richtung 204a mittels entsprechender Ansteuerung der Ventilbetätigungsspule 254 den Austrag nach Erreichen des gewünschten Austragvolumens zu unterbrechen.

Alternativ zu der Ausgestaltung mit Durchflussmesseinrichtung 280 kann eine Erhöhung der Austraggenauigkeit auch erreicht werden, indem im Speicher des Steuergeräts des Spenders Kennwerte für die spezifische Feder 258 hinterlegt sind, die zuvor im Rahmen des Herstellungsvorgangs ermittelt wurden. In gleicher Weise können bei allen drei dargestellten Ausführungsformen auch Daten in Hinblick auf einzelne Maße wie der Innendurchmesser des Ventilgehäuses 42, der Innendurchmesser des Zylinders 12, 112, 212 oder die Maße der Kanäle 72, 172 im Rahmen der Herstellung erfasst und in einem Speicher des Spenders hinterlegt werden, um sie bei der Berechnung der Parameter des Austragvorgangs zu berücksichtigen.

## Patentansprüche

1. Spender zum Austrag eines flüssigen pharmazeutischen Mediums in oder auf den Körper eines Benutzers mit
- einem Reservoir zur Aufbewahrung des Mediums,
- einer Druckerzeugungseinrichtung (10; 110; 210) zur Druckbeaufschlagung des Mediums, die als Verdrängerpumpe mit einer volumenveränderbaren Druckkammer (16; 116; 216) und einer Pumpenbetätigungseinrichtung (22, 24), mittels derer das Druckkammervolumen beeinflusst werden kann, ausgebildet ist,
- einer Auslassöffnung (44; 144; 244) zum Austragen des Mediums in oder auf den Körper des Benutzers und
- einem Auslassventil (40; 140; 240) mit einem Ventilsitz und einem zwischen einer Öffnungsstellung und einer Schließstellung relativ zum Ventilsitz beweglichen Ventilkörper (46; 146; 246),
**dadurch gekennzeichnet, dass**
- eine elektrische Ventilbetätigungseinrichtung (52, 54; 152, 154; 252, 254) vorgesehen ist, mittels derer der Ventilkörper (46; 146; 246) durch einen elektrischen Ventilsteuerstrom kraftbeaufschlagbar ist.

2. Spender nach Anspruch 1,
**dadurch gekennzeichnet, dass**
das Auslassventil (40; 240) derart ausgebildet ist, dass der Ventilkörper (46; 246) durch den Flüssigkeitsdruck des Mediums in einer dem Auslassventil zugeordneten Ventilkammer (48; 248) in Richtung (4b; 204b) seiner Öffnungsstellung kraftbeaufschlagbar ist und/oder eine dem Auslassventil (40; 140; 240) zugeordnete Ventilfedereinrichtung (58; 158; 258) vorgesehen ist, mittels derer der Ventilkörper (46; 146; 246) in Richtung (4a; 104a; 204a) seiner Schließstellung mit einer Federkraft kraftbeaufschlagbar ist.

3. Spender nach einem der vorstehenden Ansprüche,
**dadurch gekennzeichnet, dass**
die elektrische Ventilbetätigungseinrichtung (52, 54; 152, 154; 252, 254)
- eine zur Erzeugung eines Magnetfeldes durch den elektrischen Ventilsteuerstrom vorgesehene Ventilbetätigungsspule (54; 154; 254) und
- einen gegenüber der Ventilbetätigungsspule (54; 154; 254) relativbeweglichen und mittels des Magnetfeldes gegenüber der Ventilbetätigungsspule (54; 154; 254) kraftbeaufschlagbaren Gegenkörper (52; 152; 252),
aufweist, wobei
- der Gegenkörper (52; 152; 252) oder die Ventilbetätigungsspule (54; 154; 254) ortsfest zum Ventilkörper (46; 146; 246) ausgebildet ist.

4. Spender nach einem der Ansprüche 1 und 2,
**dadurch gekennzeichnet, dass**
die Ventilbetätigungseinrichtung einen durch den Ventilsteuerstrom steuerbaren Ventilpiezoaktuar zur Kraftbeaufschlagung des Ventilkörpers aufweist.

5. Spender nach einem der vorstehenden Ansprüche,
**dadurch gekennzeichnet, dass**
eine Ventilmesseinrichtung (52, 56) vorgesehen ist, die zur Erfassung einer Verlagerung des Ventilkörpers (46) relativ zum Ventilsitz ausgebildet ist, wobei die Ventilmesseinrichtung (52, 56) vorzugsweise eine Ventilmessspule (52) aufweist, die zur Erfassung der Bewegung eines am Ventilkörper (46) vorgesehenen Gegenkörpers (52) ausgebildet ist.

6. Spender nach einem der vorstehenden Ansprüche,
**dadurch gekennzeichnet, dass**
zwischen der Druckerzeugungseinrichtung (10; 110) und dem Auslassventil (40; 140) ein Durchflussbegrenzer (70; 170) vorgesehen ist, wobei der Durchflussbegrenzer (70; 170) vorzugsweise zwischen einer Druckkammer (16; 116), die der Druckerzeugungseinrichtung (10; 110) zugeordnete ist und deren Volumen durch die Druckerzeugungseinrichtung (10; 110) verminderbar ist, und einer der Auslassventil (40; 140) zugeordneten Ventilkammer (46; 146) vorgesehen ist.

7. Spender nach einem der vorstehenden Ansprüche,
**dadurch gekennzeichnet, dass**
die Pumpenbetätigungseinrichtung als manuell betätigbare Pumpenbetätigungseinrichtung ausgebildet ist, wobei die Pumpenbetätigungseinrichtung vorzugsweise ein Pumpenfedermittel (128; 228) aufweist, welches manuell spannbar ist und welches derart ausgebildet ist, dass es nach einer Auslösung im Zuge seiner Entspannung eine Volumenverringerung der Druckkammer (116; 216) bewirkt.

8. Spender nach einem der vorstehenden Ansprüche,
**dadurch gekennzeichnet, dass**
die Pumpenbetätigungseinrichtung (22, 24)
- eine zur Erzeugung eines Magnetfeldes durch einen elektrischen Pumpensteuerstrom beaufschlagbare Pumpenbetätigungsspule (24) und
- ein gegenüber der Pumpenbetätigungsspule (24) relativbewegliches und mittels des Magnetfeldes gegenüber der Pumpenbetätigungsspule (24) kraftbeaufschlagbares Gegenelement (22),
aufweist, wobei
- das Gegenelement (22) oder die Pumpenbetätigungsspule (24) derart ausgebildet und/oder angeordnet ist, dass das Druckkammervolumen durch eine Relativbewegung des Gegenelements (22) zur Pumpenbetätigungsspule (24) veränderbar ist.

9. Spender nach einem der vorstehenden Ansprüche,
**dadurch gekennzeichnet, dass**
eine Pumpenmesseinrichtung (22, 26) vorgesehen ist, die zur Erfassung des Zustandes der Pumpenbetätigungseinrichtung (22, 24) ausgebildet ist, wobei vorzugsweise die die Pumpenmesseinrichtung (22, 26) eine Pumpenmessspule (22) aufweist, die zur Erfassung der Lage eines der Pumpenbetätigungseinrichtung (22, 24) zugeordneten Bauteils (22) ausgebildet ist, welches zum Zwecke der Beeinflussung des Druckkammervolumens lageveränderlich ist.

10. Spender nach einem der vorstehenden Ansprüche,
**dadurch gekennzeichnet, dass**
in der Druckkammer (116) und/oder in der Ventilkammer (146) ein Drucksensor (130, 160) vorgesehen ist und/oder ein Durchflusssensor (280) vorgesehen ist, der vorzugsweise zwischen der Druckkammer (216) und der Ventilkammer (246) angeordnet ist.

11. Spender nach einem der vorstehenden Ansprüche,
**gekennzeichnet durch**
ein Steuergerät, **durch** das der Ventilsteuerstrom und/oder der Pumpensteuerstrom steuerbar sind und/oder **durch** das Ausgangssignale der Pumpenmesseinrichtung (22, 26) und/oder der Ventilmesseinrichtung (52, 56) auswertbar sind.

12. Verfahren zum Austragen eines flüssigen pharmazeutischen Mediums mittels eines Spenders nach einem der vorstehenden Ansprüche,
**gekennzeichnet durch**
einen Austragvorgang mit den Schritten:
a. Das Medium wird innerhalb einer Druckkammer (16; 116; 216) einer Druckerzeugungseinrichtung mit einem Druck p1 beaufschlagt,
b. Ein Auslassventil (40; 140; 240) wird zum Zwecke des Medienaustrags geöffnet und
c. Das Auslassventil (40; 140; 240) wird nach einer Öffnungszeit Δt **durch** ein elektrisches Steuersignal wieder geschlossen, um den Medienaustrag zu beenden.

13. Verfahren nach Anspruch 12,
**dadurch gekennzeichnet, dass**
das Auslassventil (40) in Schritt b bei Erreichen eines vorgegebenen Öffnungsdruck p2 des Mediums, der geringer ist als der Druck p1, unmittelbar durch diesen Öffnungsdruck p2 geöffnet wird.

14. Verfahren nach einem der Ansprüche 12 oder 13,
**dadurch gekennzeichnet, dass**
die Schritte b und c wiederholt werden, bis ein Austragvolumen und/oder eine Anzahl an Zyklen erreicht ist, wobei die Wiederholung vorzugsweise mit einer Frequenz >= 20 Hz, insbesondere >= 100 Hz, besonders bevorzugt >= 1000 Hz erfolgt.

15. Verfahren nach einem der Ansprüche 12 bis 14,
**dadurch gekennzeichnet, dass**
dem Austragvorgang
- ein Messvorgang zur Erfassung von auf einen Ventilkörper (46) des Austragventils (40) und/oder auf einen Kolben (14) der Druckerzeugungsvorrichtung (10) wirkenden Kräften vorausgeht und/oder
- ein Erstbefüllungsvorgang vorausgeht, bei dem nach Öffnen des Auslassventils (40; 140; 240) durch elektrisches Steuersignal vorhandene Luft aus dem Strömungspfad zwischen der Druckkammer (16; 116; 216) und einer Auslassöffnung (44; 144; 244) mittels der Druckerzeugungseinrichtung (10; 110; 210) verdrängt wird.

16. Verfahren nach einem der Ansprüche 12 bis 15,
**dadurch gekennzeichnet, dass**
die Öffnungszeit Δt sowie vorzugsweise auch die Anzahl der Zyklen vor Beginn des Austragvorgangs festgelegt sind.

17. Herstellungsverfahren zur Herstellung eines Spenders zum Austrag eines flüssigen pharmazeutischen Mediums in oder auf dem Körper eines Benutzers, zur Herstellung eines Spenders nach einem der Ansprüche 1 bis 11,
**dadurch gekennzeichnet, dass**
spezifische Parameter der Austragvorrichtung im Zuge der Herstellung gemessen oder ermittelt werden und in einem Speicher der Austragvorrichtung gespeichert werden.

## Claims

1. Discharge device for discharging a liquid pharmaceutical medium into or onto the body of a user, having
- a reservoir for storing the medium,
- a pressure generator (10; 110; 210) for pressurizing the medium, which generator is constructed as a positive displacement pump with a volume-variable pressure chamber (16; 116; 216) and a pump actuator (22, 24) by means of which it is possible to influence the pressure chamber volume,
- a discharge opening (44; 144; 244) for discharging the medium into or onto the body of a user, and
- an outlet valve (40; 140; 240) with a valve seat and a valve body (46; 146; 246) movable relative to the valve seat between an open position and a closed position,
**characterized in that**
- an electrical valve actuating device (52, 54; 152, 154; 252, 254) is provided by means of which a force can be applied to the valve body (46; 146; 246) by an electrical valve control current.

2. Discharge device according to claim 1, **characterized in that** the discharge valve (40; 240) is constructed in such a way that the valve body (46; 246) can be forced in the direction (4b; 204b) of its open position by the fluid pressure of the medium in a valve chamber (48; 248) associated with the discharge valve and/or a valve spring mechanism (58; 158; 258) associated with the discharge valve (40; 140; 240) is provided and by means thereof a spring force can be applied to the valve body (46; 146; 246) in the direction (4a; 104a; 204a) of its closed position.

3. Discharge device according to any one of the preceding claims, **characterized in that** the electrical valve actuating device (52, 54; 152, 154; 252, 254) has
- a valve actuating coil (54; 154; 254) provided for producing a magnetic field through the electrical valve control current and
- a counterbody (52; 152; 252) movable relative to the valve actuating coil (54; 154; 254) and to which a force can be applied by means of the magnetic field with respect to the valve actuating coil (54; 154; 254),
wherein
- the counterbody (52; 152; 252) or the valve actuating coil (54; 154; 254) is fixed relative to the valve body (46; 146; 246).

4. Discharge device according to any one of the claims 1 and 2, **characterized in that** the valve actuating device has a valve piezoactuator controllable by the valve control current for applying a force to the valve body.

5. Discharge device according to any one of the preceding claims, **characterized in that** a valve measuring device (52, 56) is provided and is constructed for detecting a displacement of the valve body (46) relative to the valve seat, wherein the valve measuring device (52, 56) preferably has a valve measuring coil (52) constructed for detecting movement of a counterbody (52) provided on the valve body (46).

6. Discharge device according to any one of the preceding claims, **characterized in that** a flow limiter (70; 170) is provided between the pressure generator (10; 110) and the discharge valve (40; 140), wherein the flow limiter (70; 170) preferably is located between a pressure chamber (16; 116) associated with the pressure generator (10; 110) and whose volume can be reduced by said pressure generator (10; 110) and a valve chamber (46; 146) associated with the discharge valve (40; 140).

7. Discharge device according to any one of the preceding claims, **characterized in that** the pump actuator is constructed as a manually actuatable pump actuator, the pump actuator preferably having a pump spring means (128; 228) which can be manually tensioned and which is constructed in such a way that, following release during its relaxation, it brings about a volume reduction of the pressure chamber (116; 216).

8. Discharge device according to any one of the preceding claims, **characterized in that** the pump actuator (22, 24) has
- a pump actuating coil (24) to which a force can be applied for producing a magnetic field through an electrical pump control current and
- a counterelement (22) relatively movable in relation to the pump actuating coil (24) and to which a force can be applied by means of the magnetic field with respect to the pump actuating coil (24),
wherein
- the counterelement (22) or the pump actuating coil (24) is constructed and/or positioned such that the pressure chamber volume can be varied through a relative movement of the counterelement (22) to the pump actuating coil (24).

9. Discharge device according to any one of the preceding claims, **characterized in that** a pump measuring device (22, 26) is provided and is constructed for detecting the state of the pump actuator (22, 24), wherein preferably the pump measuring device (22, 26) has a pump measuring coil (22) constructed for detecting the position of a component (22) associated with the pump actuator (22, 24) and whose position can be varied for the purpose of influencing the pressure chamber volume.

10. Discharge device according to any one of the preceding claims, **characterized in that** within the pressure chamber (116) and/or within the valve chamber (146) a pressure sensor (130, 160) is provided and/or a flow sensor (280) is provided, preferably positioned between the pressure chamber (216) and the valve chamber (246).

11. Discharge device according to any one of the preceding claims, **characterized by** a control unit permitting control of the valve control current and/or pump control current and/or permitting evaluation of the output signals of the pump measuring device (22, 26) and/or valve measuring device (52, 56).

12. Method for discharging a liquid pharmaceutical medium by means of a discharge device according to any one of the preceding claims, **characterized by** a discharge process with the steps:
a. within a pressure chamber (16; 116; 216) a pressure p1 is applied by a pressure generator to the medium,
b. a discharge valve (40; 140; 240) is opened for medium discharge purposes and
c. following an opening time Δt, the discharge valve (40; 140; 240) is closed again by an electrical control signal in order to terminate medium discharge.

13. Method according to claim 12, **characterized in that** in step b, on reaching a preset medium opening pressure p2 lower than the pressure p1, the discharge valve (40) is directly opened by said opening pressure p2.

14. Method according to claim 12 or 13, **characterized in that** steps b and c are repeated until a discharge volume and/or a number of cycles is reached, the repetition preferably taking place with a frequency > = 20 Hz, particularly > = 100 Hz and with particular preference > = 1000 Hz.

15. Method according to any one of claims 12 to 14, **characterized in that** the discharge process
- is preceded by a measuring process for detecting forces acting on a valve body (46) of the discharge valve (40) and/or on a piston (14) of the pressure generator (10) and/or
- is preceded by an initial filling process during which, following the opening of the discharge valve (40; 140; 240) by an electrical control signal, air is displaced from the flow path between the pressure chamber (16; 116; 216) and a discharge opening (44; 144; 244) by means of the pressure generator (10; 110; 210).

16. Method according to any one of claims 12 to 15, **characterized in that** the opening time Δt and preferably also the number of cycles are fixed prior to the start of the discharge process.

17. Manufacturing method for producing a discharge device for discharging a liquid pharmaceutical medium into or onto the body of a user, for producing a discharge device according to any one of the claims 1 to 11,
**characterized in that**
specific parameters of the discharge device are measured or determined during manufacture and stored in a memory of the discharge device.

## Revendications

1. Distributeur pour la sortie d'un fluide pharmaceutique liquide dans ou sur le corps d'un utilisateur, avec
- un réservoir pour la conservation du fluide,
- un dispositif de production de pression (10; 110; 210) pour la mise sous pression du fluide, qui est configuré en forme de pompe de refoulement avec une chambre de pression (16; 116; 216) à volume variable et un dispositif d'actionnement de pompe (22, 24) au moyen duquel le volume de la chambre de pompe peut être influencé,
- une ouverture de sortie (44; 144; 244) pour la sortie du fluide dans ou sur le corps de l'utilisateur, et
- une soupape de sortie (40; 140; 240) avec un siège de soupape et un corps de soupape (46; 146; 246) mobile entre une position ouverte et une position fermée par rapport au siège de soupape,
**caractérisé en ce que**
- il est prévu un dispositif électrique d'actionnement de soupape (52, 54; 152, 154; 252, 254), au moyen duquel le corps de soupape (46; 146; 246) peut être soumis à une force par un courant électrique de commande de soupape.

2. Distributeur selon la revendication 1, **caractérisé en ce que** la soupape de sortie (40; 240) est configurée de telle manière que le corps de soupape (46; 246) puisse être soumis à une force par la pression de liquide du fluide dans une chambre de soupape (48; 248) associée à la soupape de sortie en direction (4b; 204b) de sa position ouverte et/ou **en ce qu'**il est prévu un dispositif de ressort de soupape (58; 158; 258) associé à la soupape de sortie (40; 140; 240), au moyen duquel le corps de soupape (46; 146; 246) peut être soumis à une force en direction de sa position fermée au moyen d'une force de ressort.

3. Distributeur selon l'une quelconque des revendications précédentes, **caractérisé en ce que** le dispositif électrique d'actionnement de soupape (52, 54; 152, 154; 252, 254) présente
- une bobine d'actionnement de soupape (54; 154; 254) prévue pour la production d'un champ magnétique par le courant électrique de commande de soupape, et
- un corps opposé (52; 152; 252) mobile relativement par rapport à la bobine d'actionnement de soupape (54; 154; 254) et pouvant être soumis à une force au moyen du champ magnétique par rapport à la bobine d'actionnement de soupape (54; 154; 254),
dans lequel
- le corps opposé (52; 152; 252) ou la bobine d'actionnement de soupape (54; 154; 254) est immobile par rapport au corps de soupape (46; 146; 246).

4. Distributeur selon une des revendications 1 et 2, **caractérisé en ce que** le dispositif d'actionnement de soupape présente un actionneur piézoélectrique de soupape pouvant être commandé par le courant de commande de soupape pour soumettre le corps de soupape à une force.

5. Distributeur selon l'une quelconque des revendications précédentes, **caractérisé en ce qu'**il est prévu un dispositif de mesure de soupape (52, 56), qui est configuré de façon à détecter un déplacement du corps de soupape (46) par rapport au siège de soupape, dans lequel le dispositif de mesure de soupape (52, 56) présente de préférence une bobine de mesure de soupape (52), qui est configurée de façon à détecter le mouvement d'un corps opposé (52) prévu sur le corps de soupape (46).

6. Distributeur selon l'une quelconque des revendications précédentes, **caractérisé en ce qu'**il est prévu entre le dispositif de production de pression (10; 110) et la soupape de sortie (40; 140) un limiteur de débit (70; 170), dans lequel le limiteur de débit (70; 170) est prévu de préférence entre une chambre de pression (16; 116), qui est associée au dispositif de production de pression (10; 110) et dont le volume peut être réduit par le dispositif de production de pression (10; 110), et une chambre de soupape (46; 146) associée à la soupape de sortie (40; 140).

7. Distributeur selon l'une quelconque des revendications précédentes, **caractérisé en ce que** le dispositif d'actionnement de pompe est configuré en forme de dispositif d'actionnement de pompe actionnable manuellement, dans lequel le dispositif d'actionnement de pompe présente de préférence un moyen de ressort de pompe (128; 228), qui peut être contraint manuellement et qui est configuré de telle manière qu'il provoque une diminution de volume de la chambre de pression (116; 216) après un déclenchement à la suite de sa détente.

8. Distributeur selon l'une quelconque des revendications précédentes, **caractérisé en ce que** le dispositif d'actionnement de pompe (22, 24) présente
- une bobine d'actionnement de pompe (24) pouvant être soumise à un courant électrique de commande de pompe pour la production d'un champ magnétique et
- un élément opposé (22) mobile relativement par rapport à la bobine d'actionnement de pompe (24) et pouvant être soumis à une force au moyen du champ magnétique par rapport à la bobine d'actionnement de pompe (24),
dans lequel
- l'élément opposé (22) ou la bobine d'actionnement de pompe (24) est configuré(e) et/ou disposé (e) de telle manière que le volume de la chambre de pression puisse être modifié par un mouvement relatif de l'élément opposé (22) par rapport à la bobine d'actionnement de pompe (24).

9. Distributeur selon l'une quelconque des revendications précédentes, **caractérisé en ce qu'**il est prévu un dispositif de mesure de pompe (22, 26), qui est configuré de façon à détecter l'état du dispositif d'actionnement de pompe (22, 24), dans lequel le dispositif de mesure de pompe (22, 26) présente de préférence une bobine de mesure de pompe (22), qui est configurée de façon à détecter la position d'un composant (22) associé au dispositif d'actionnement de pompe (22, 24), qui peut changer de position dans le but d'influencer le volume de la chambre de pompe.

10. Distributeur selon l'une quelconque des revendications précédentes, **caractérisé en ce qu'**il est prévu un détecteur de pression (130, 160) dans la chambre de pompe (116) et/ou dans la chambre de soupape (146) et/ou **en ce qu'**il est prévu un détecteur de débit (280), qui est disposé de préférence entre la chambre de pression (216) et la chambre de soupape (246).

11. Distributeur selon l'une quelconque des revendications précédentes, **caractérisé par** un appareil de commande, par lequel le courant de commande de soupape et/ou le courant de commande de pompe peuvent être commandés et/ou par lequel des signaux de sortie du dispositif de mesure de pompe (22, 26) et/ou du dispositif de mesure de soupape (52, 56) peuvent être exploités.

12. Procédé pour faire sortir un fluide pharmaceutique liquide au moyen d'un distributeur selon l'une quelconque des revendications précédentes, **caractérisé par** une opération de sortie comprenant les étapes suivantes:
a. on soumet le fluide à une pression p1 à l'intérieur d'une chambre de pression (16; 116; 216) d'un dispositif de production de pression,
b. on ouvre une soupape de sortie (40; 140; 240) dans le but de faire sortir le fluide, et
c. on ferme de nouveau la soupape de sortie (40; 140; 240) après une durée d'ouverture Δt, au moyen d'un signal de commande électrique, afin de mettre fin à la sortie de fluide.

13. Procédé selon la revendication 12, **caractérisé en ce que** l'on ouvre la soupape de sortie (40) à l'étape b lorsque l'on atteint une pression d'ouverture prédéterminée p2, qui est inférieure à la pression p1, directement au moyen de cette pression d'ouverture p2.

14. Procédé selon l'une des revendications 12 ou 13, **caractérisé en ce que** l'on répète les étapes b et c, jusqu'à ce qu'un volume de sortie et/ou un nombre de cycles soit atteint, dans lequel la répétition est opérée de préférence avec une fréquence ≥ 20 Hz, en particulier ≥ 100 Hz, de préférence encore ≥ 1000 Hz.

15. Procédé selon l'une quelconque des revendications 12 à 14, **caractérisé en ce que** l'opération de sortie est précédée par
- une opération de mesure destinée à détecter des forces agissant sur un corps de soupape (46) de la soupape de sortie (40) et/ou sur un piston (14) du dispositif de production de pression (10), et/ou
- une première opération de remplissage, dans laquelle, après l'ouverture de la soupape de sortie (40; 140; 240) par un signal de commande électrique, l'air présent est chassé hors du chemin d'écoulement entre la chambre de pression (16; 116; 216) et une ouverture de sortie (44; 144; 244) au moyen du dispositif de production de pression (10; 110; 210).

16. Procédé selon l'une quelconque des revendications 12 à 15, **caractérisé en ce que** l'on fixe la durée d'ouverture Δt ainsi que de préférence aussi le nombre des cycles avant le commencement de l'opération de sortie.

17. Procédé de fabrication pour la fabrication d'un distributeur pour la sortie d'un fluide pharmaceutique liquide dans ou sur le corps d'un utilisateur, pour la fabrication d'un distributeur selon l'une quelconque des revendications 1 à 11, **caractérisé en ce que** l'on mesure ou on détermine des paramètres spécifiques du dispositif de sortie au cours de la fabrication et on les mémorise dans une mémoire du dispositif de sortie.
